(19) Europäisches Patentamt
European Patent Office
Office européen des brevets

(11) Numéro de publication: **0 433 163 A2**

# (12) DEMANDE DE BREVET EUROPEEN

(21) Numéro de dépôt: **90403526.8**

(22) Date de dépôt: **11.12.90**

(51) Int. Cl.[5]: **C07D 487/04**, A61K 31/55, C07D 233/64, C07D 233/90, //(C07D487/04,243:00,235:00)

(30) Priorité: **11.12.89 GB 8927928**

(43) Date de publication de la demande: **19.06.91 Bulletin 91/25**

(84) Etats contractants désignés: **AT BE CH DE DK ES FR GB GR IT LI LU NL SE**

(71) Demandeur: **ROUSSEL-UCLAF**
**35, boulevard des Invalides**
**F-75007 Paris(FR)**

(72) Inventeur: **Hedgecock, Charles John Robert**
**186 High Street**
**Wootton Basset, Wiltshire SN4 7BZ(GB)**
Inventeur: **Gardner, Colin Robert**
**Foxlea, Hermitage Road**
**Cold Ash, Newbury, Berks(GB)**
Inventeur: **Jones, Stuart John**
**1 Walnut Tree Gardens, Lydiard Millicent**
**Swindon, Wiltshire SN5 9LH(GB)**

(74) Mandataire: **Vieillefosse, Jean-Claude et al**
**Roussel-Uclaf 111, route de Noisy B.P. 9**
**F-93230 Romainville(FR)**

## (54) Nouveau procédé de préparation d'imidazobenzodiazépines et de leurs sels.

(57) L'invention concerne la préparation d'imidazobenzodiazépines :

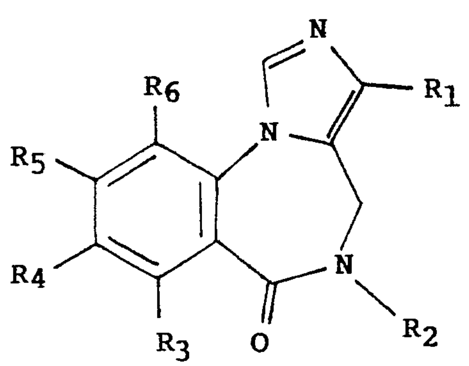

(I)

dans laquelle :

$R_2$ représente hydrogène, alkyle, cycloalkyle, phényle éventuellement substitué,

$R_3$, $R_4$ représentent hydrogène, halogène, $CX_3$ (X étant halogène), nitro, cyano, NRR', un groupement CONRR' (R étant hydrogène ou alkyle et R' étant alkyle ou R et R' étant alkylène), $OR_7$, $SR_7$, $COOR_7$ ou $COR_7$ ($R_7$ étant hydrogène, alkyle phényle,

$R_5$ représente hydrogène ou fluor et :

- soit $R_1$ représente :

EP 0 433 163 A2

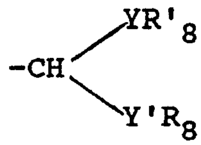

($R_8$ et $R'_8$ étant alkyle ou ensemble alkylène et Y et Y' étant oxygène ou soufre et $R_6$ représente hydrogène, halogène, nitro, amino, -NRR', $SR_7$, $OR_7$ ou $COR_7$,

- soit $R_1$ représente $COOR_9$ ($R_9$ étant alkyle) ou cycloalkyle et $R_6$ représente hydrogène, halogène, nitro, NRR', CONRR', $-OR_7$, $SR_7$, $COOR_7$, $COR_7$, $-N=CH-OR_9$, CN, amino, $-N=CH-N(CH_3)_2$ ou thiocyano, isothiocyano ou thioacétyle,
- soit $R_1$ représente $-COR'_c$ ($R'_c$ étant cycloalkyle) et $R_6$ hydrogène, halogène, nitro, -NRR' ou -CONRR', $-SR_7$, $-OR_7$, $-COOR_7$, $-COR_7$, $-N=CH-OR_9$, CN, et de leurs sels ainsi que des nouveaux produits obtenus par ce procédé.

## NOUVEAU PROCÉDÉ DE PRÉPARATION D'IMIDAZOBENZODIAZÉPINES ET DE LEURS SELS.

La présente invention concerne un nouveau procédé de préparation d'imidazobenzodiazépines ainsi que de leurs sels.

Dans sa demande de brevet européen n° 0 305 298, la demanderesse a décrit de nouvelles imidazobenzodiazépines et leurs sels, le procédé de préparation et l'application à titre de médicaments de ces nouveaux produits. Lors de l'étude de ceux-ci, la demanderesse a été amenée à étudier le procédé de préparation de ces derniers et a trouvé de manière tout à fait inattendue qu'il était possible d'obtenir les produits recherchés en 2 à 3 stades seulement avec un rendement beaucoup plus important.

Le nouveau procédé permet d'obtenir également des imidazobenzodiazépines qui ne sont pas accessibles par les procédés décrits antérieurement.

La présente demande a ainsi pour objet un nouveau procédé de préparation d'imidazobenzodiazépines répondant à la formule générale (I) :

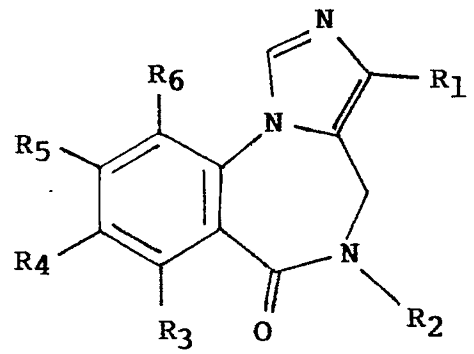

(I)

dans laquelle :

$R_2$ représente un atome d'hydrogène, un radical alkyle renfermant de 1 à 5 atomes de carbone, un radical cycloalkyle renfermant de 3 à 6 atomes de carbone, un groupement phényle, un groupement phényle substitué,

$R_3$, $R_4$, identiques ou différents, représentent un atome d'hydrogène, un atome d'halogène, un groupement $CX_3$ dans lequel X représente un atome d'halogène, un groupement $NO_2$, un groupement CN, un groupement NRR', un groupement CONRR', dans lesquels R représente un atome d'hydrogène ou un radical alkyle renfermant de 1 à 5 atomes de carbone et R' représente un radical alkyle renfermant de 1 à 5 atomes de carbone, ou R et R' ensemble représentent un radical alkylène, renfermant de 2 à 5 atomes de carbone, soit $R_3$ et $R_4$ représentent un radical $OR_7$, $SR_7$, $COOR_7$ ou $COR_7$ dans lequel $R_7$ représente un atome d'hydrogène, un radical alkyle renfermant de 1 à 5 atomes de carbone ou un groupement phényl,

$R_5$ représente un atome d'hydrogène ou de fluor et que :

- soit $R_1$ représente un groupement :

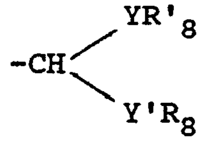

dans lequel $R_8$ et $R'_8$ identiques ou différents représentent un radical alkyle renfermant de 1 à 3 atomes de carbone ou $R_8$ et $R'_8$ ensemble représentent un groupe alkylène contenant de 2 à 5 atomes de carbone et Y et Y' identiques ou différents, représentent un atome d'oxygène ou un atome de soufre, et $R_6$ représente un atome d'hydrogène, un atome d'halogène, un groupement nitro, un groupement amino, un groupement -NRR' dans lequel R et R' ont la signification déjà indiquée ou $R_6$ représente un groupement $SR_7$, un groupement $OR_7$, un groupement $COR_7$, groupements dans lesquels $R_7$ a la signification déjà indiquée,

- soit $R_1$ représente un groupement $COOR_9$ dans lequel $R_9$ représente un radical alkyle renfermant de 1 à 5 atomes de carbone ou $R_1$ représente un groupement cycloalkyle renfermant de 3 à 6 atomes

de carbone, et $R_6$ représente un atome d'hydrogène, un atome d'halogène, un groupement nitro, un groupement NRR', un groupement CONRR' dans lesquels R et R' ont la signification déjà indiquée, un radical $-OR_7$, $SR_7$, $COOR_7$ ou $COR_7$ dans lesquels $R_7$ a la signification déjà indiquée, ou un groupement $-N=CH-OR_9$ dans lequel $R_9$ a la signification déjà indiquée, ou $R_6$ représente un groupement CN, un groupement amino, un groupement $-N=CH-N(CH_3)_2$ ou un groupement thiocyano, isothiocyano ou thioacétyle,

- soit $R_1$ représente un groupement $-COR'_c$ dans lequel $R'_c$ représente un groupement cycloalkyle renfermant de 3 à 6 atomes de carbone et $R_6$ représente un atome d'hydrogène, un atome d'halogène, un groupement nitro, un groupement -NRR' ou -CONRR' dans lesquels R et R' ont la signification déjà indiquée, ou un groupement $-SR_7$, $-OR_7$, $-COOR_7$ ou $-COR_7$ dans lesquels $R_7$ a la signification déjà indiquée, ou un groupement $-N=CH-OR_9$ dans lequel $R_9$ a la signification déjà indiquée, ou un groupement CN, ainsi que de leurs sels d'addition avec les acides.

Le procédé selon l'invention est caractérisé en ce que :

1) pour préparer des produits de formule ($I_A$) :

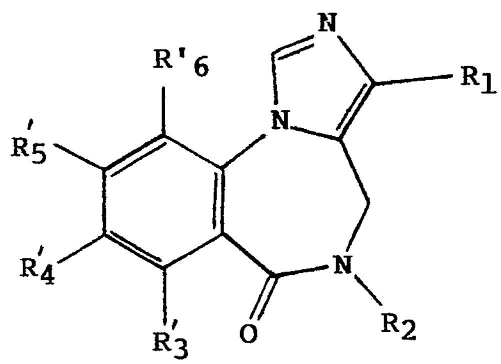

($I_A$)

dans laquelle $R'_3$, $R'_4$, $R'_5$ et $R'_6$ ont la signification indiquée ci-dessus pour $R_3$, $R_4$, $R_5$ et $R_6$ à la condition que l'un au moins de $R'_3$, $R'_4$, $R'_5$ et $R'_6$ soit un groupe éléctro attracteur, et $R_1$ et $R_2$ ont la signification déjà indiquée, on fait réagir un produit de formule (II) :

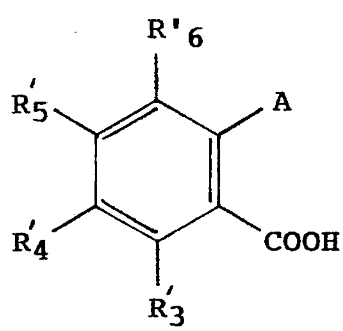

(II)

dans laquelle $R'_3$, $R'_4$, $R'_5$ et $R'_6$ ont la signification déjà indiquée, A représente un groupement éliminable ou un dérivé fonctionnel de cet acide de formule (II) avec un produit de formule (III) :

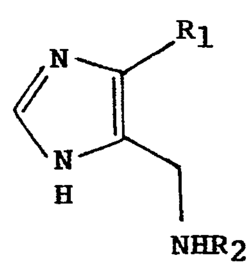

(III)

dans laquelle $R_1$ et $R_2$ ont la signification déjà indiquée, pour obtenir un produit de formule (IV) :

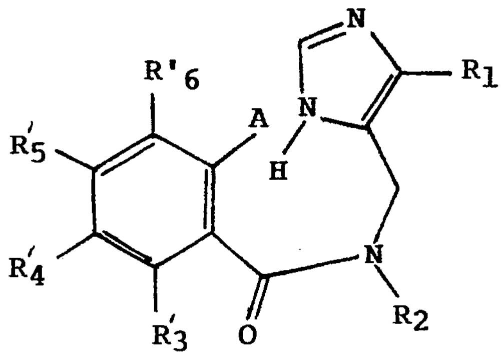

(IV)

dans laquelle $R_1$, $R_2$, $R'_3$, $R'_4$, $R'_5$, $R'_6$ et A ont la signification déjà indiquée, puis cyclise ce dernier et si désiré, salifie le produit de formule ($I_A$) obtenu.

Dans des conditions préférentielles de mise en oeuvre de l'invention, le procédé de préparation ci-dessus décrit est caractérisé en ce que :

- le groupement éliminable A est constitué par un atome d'halogène de préférence un atome de chlore, de brome ou de fluor ou par un groupement nitro,
- $R'_3$ et $R'_4$ identiques ou différents représentent un atome d'hydrogène, un atome d'halogène, un groupe trifluorométhyl ou nitro,
- $R'_6$ représente un atome d'hydrogène, un atome d'halogène ou un groupe nitro,
- le dérivé fonctionnel de l'acide de formule (II) est avantageusement le chlorure d'acide,
- la réaction du produit de formule (II) avec le produit de formule (III) est avantageusement effectuée au sein d'un solvant organique tel que le dichlorométhane en présence d'un agent alcalin tel que le carbonate de sodium l'hydroxyde de sodium, de potassium ou d'ammonium, ou en présence d'une amine telle que la triméthylamine ou la pyridine,
- la cyclisation du produit de formule (IV) est avantageusement effectuée en présence d'hydrure de lithium au sein d'un solvant organique anhydre tel que le diméthylformamide, au reflux du mélange réactionnel,

2) pour préparer des produits de formule (I) telle que définie ci-dessus, l'on fait réagir un produit de formule ($I_B$) :

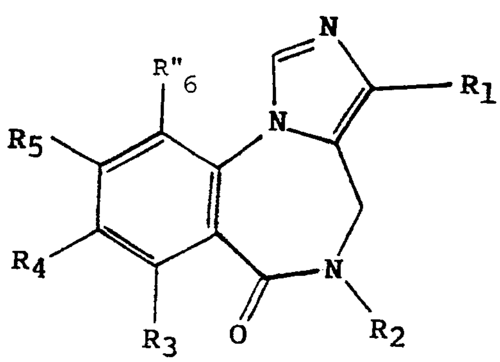

($I_B$)

dans laquelle $R_1$, $R_2$, $R_3$, $R_4$ et $R_5$ ont la signification déjà indiquée et $R''_6$ a l'une des significations indiquées ci-dessus pour $R_6$ à l'exception du groupement $R_6$ désiré, avec un réactif approprié capable de remplacer le groupement $R''_6$ par le groupement $R_6$, et si désiré salifie le produit de formule (I) obtenu.

Lorsque $R''_6$ représente un groupement électro-attracteur, le produit de départ de formule ($I_B$) peut être préparé comme indiqué au point 1) et correspond au produit de formule ($I_A$).

Selon le point 2), le groupement électro-attracteur, $R''_6$ peut être remplacé par un groupement non électro-attracteur $R_6$. Cette méthode permet ainsi de conduire aux produits de formule (I) dans laquelle aucun des groupements $R_3$, $R_4$, $R_5$ ou $R_6$ n'est électro-attracteur.

Lorsque l'on désire préparer un produit de formule (I) dans laquelle $R_6$ représente un groupement $NH_2$, on effectue une réduction sur un produit de formule ($I_B$) dans laquelle $R''_6$ représente un groupe $NO_2$.

La réduction du groupement $NO_2$ en groupement $NH_2$ est effectuée au moyen d'un réducteur doux en présence d'un catalyseur ; la réduction peut avantageusement être effectuée au sein d'un mélange méthanol/dioxane au reflux du milieu réactionnel en présence d'un catalyseur à base de palladium et de formate d'ammonium.

Lorsque l'on désire préparer un produit de formule (I) dans lequel $R_6$ représente un groupement $-N=CH-O-R_9$, on fait avantageusement réagir un produit de formule ($I_B$) dans lequel $R''_6$ représente un groupement $NH_2$ avec un trialkyl orthoformate au reflux du mélange réactionnel.

Lorsque l'on désire préparer un produit de formule (I) dans lequel $R_6$ représente un groupement NRR' on peut avantageusement réduire un produit de formule ($I_B$) dans lequel $R''_6$ représente un groupement $-N=CH-O-R_9$ au moyen du borohydrure de sodium.

Lorsque l'on désire préparer un produit de formule (I) dans lequel $R_6$ représente un groupement $-SR_7$, on peut avantageusement faire réagir un produit de formule ($I_B$) dans lequel $R''_6$ représente un groupement $NH_2$ avec du butyl nitrite puis avec un produit de formule $R_7$-S-Cu dans laquelle $R_7$ a la signification indiquée ci-dessus.

3) Pour préparer des produits de formule ($I_C$) :

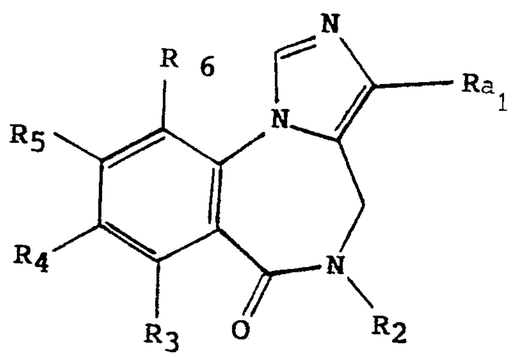

($I_C$)

dans laquelle $R_2$, $R_3$, $R_4$, $R_5$ et $R_6$ ont la signification déjà indiquée et $Ra_1$ représente un groupe $-COR'_c$ dans lequel $R'_c$ a la signification déjà indiquée, l'on fait réagir un produit de formule (VIII) :

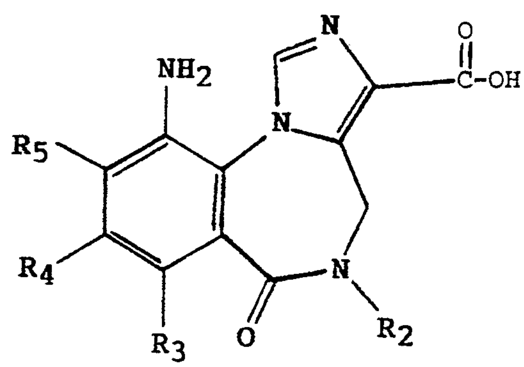

(VIII)

dans laquelle $R_2$, $R_3$, $R_4$ et $R_5$ ont la signification déjà indiquée, avec le 1,1-carbonyldiimidazole pour obtenir un produit de formule (VII) :

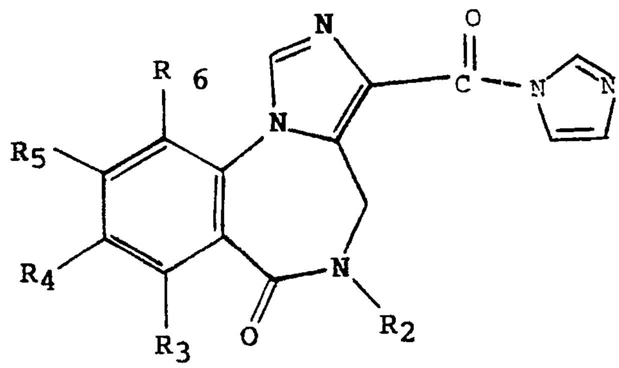

(VII)

dans laquelle $R_2$, $R_3$, $R_4$, $R_5$ et $R_6$ ont la signification déjà indiquée, que l'on fait réagir avec le chlorhydrate de N,O-diméthylhydroxylamine pour obtenir un produit de formule (V) :

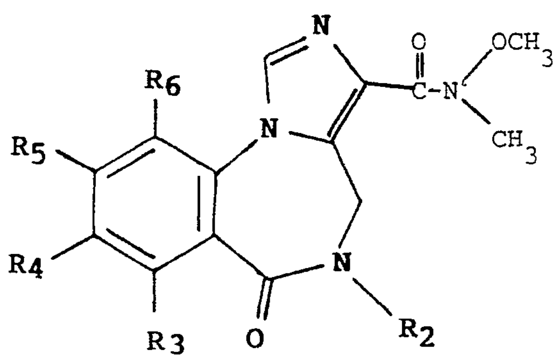

(V)

dans laquelle $R_2$, $R_3$, $R_4$, $R_5$ et $R_6$ ont la signification déjà indiquée, que l'on fait réagir avec un produit de formule (VI) :

$$R'_c\text{-Mg-Hal} \qquad (VI)$$

dans laquelle $R'_c$ a la signification déjà indiquée et Hal représente un atome d'halogène et si désiré, salifie le produit de formule ($I_C$) obtenu.

Dans des conditions préférentielles de mise en oeuvre de l'invention, le procédé de préparation ci-dessus décrit est caractérisé en ce que :

- la réaction du produit de formule (VIII) avec le 1,1-carbonyldiimidazole et la réaction du produit de formule (VII) avec le chlorhydrate de N,O-diméthylhydroxylamine sont avantageusement effectuées au sein d'un solvant organique anhydre tel que le chlorure de méthylène,
- la réaction du produit de formule(V) avec le produit de formule (VI) est avantageusement effectuée au sein d'un solvant organique anhydre tel que le tétrahydrofuranne.

Dans la formule générale (I) et dans ce qui précède :

- par radical alkyle renfermant de 1 à 5 atomes de carbone, on entend de préférence un radical méthyle, éthyle, propyle, isopropyle, butyle, isobutyle, tert-butyle ou pentyle,
- par radical alcoyle renfermant de 1 à 3 atomes de carbone, on entend un radical méthyle, éthyle, propyle, isopropyle,
- par groupement phényle substitué on entend de préférence un groupement phényle substitué par un atome d'halogène, un radical alkyle renfermant de 1 à 3 atomes de carbone, un radical alcoxy renfermant de 1 à 3 atomes de carbone, un groupement trifluorométhyle,
- par atome d'halogène, on entend de préférence un atome de fluor, de chlore, de brome ou d'iode,
- par groupement NRR' dans lequel R et R' ont la signification déjà indiquée, on entend de préférence un radical mono ou dialkylamino tel que les radicaux monométhylamino, diméthylamino, monoéthylamino, diéthylamino, monopropylamino, dipropylamino, méthyléthylamino, méthylpropylamino ou mé-

thylisopropylamino,

- par groupement $OR_7$ dans lequel $R_7$ a la signification déjà indiquée, on entend de préférence un radical méthoxy, éthoxy, propoxy, isopropoxy ou phénoxy,
- par groupement $SR_7$ dans lequel $R_7$ a la signification déjà indiquée, on entend de préférence un radical méthylthio, éthylthio, propylthio, isopropylthio ou phénylthio,
- par groupement CONRR' dans lequel R et R' ont la signification déjà indiquée, on entend de préférence un radical monométhylamido, diméthylamido, monoéthylamido, diéthylamido, monopropylamido, dipropylamido,
- par groupement cycloalkyle renfermant de 3 à 6 atomes de carbone, on entend de préférence un radical cyclopropyle, cyclobutyle, cyclopentyle ou cyclohexyle,
- par groupement alkylène contenant de 2 à 5 atomes de carbone, on entend de préférence un groupement éthylène, triméthylène, propylène, tétraméthylène ou pentaméthylène,
- par groupement électro-attracteur, on entend par exemple un atome de fluor, de chlore ou de brome, un groupement nitro ou trifluorométhyle.

Les produits de formule (I) présentent un caractère basique.

On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les sels d'addition avec les acides minéraux ou organiques peuvent être, par exemple, les sels formés avec les acides chlorhydrique, bromhydrique, iodhydrique, nitrique, sulfurique, phosphorique, propionique, formique, benzoïque, maléïque, fumarique, succinique, tartrique, citrique, oxalique, glyoxylique, aspartique, alcanesulfoniques tel que l'acide méthanesulfonique et arylsulfoniques tel que l'acide benzènesulfonique.

L'invention a également pour objet à titre de produits nouveaux les imidazobenzodiazépines appelées produits de formule ($I_D$) dont les noms suivent :

- 5,6-dihydro-5-méthyl-10-nitro-6-oxo-4H-imidazo [1,5-a] [1,4] benzodiazépine-3-carboxylate d'éthyle,
- 5,6-dihydro-10-fluoro-5-méthyl-6-oxo-4H-imidazo [1,5-a] [1,4] benzodiazépine-3-carboxylate d'éthyle,
- 7,10-difluoro-5,6-dihydro-5-méthyl-6-oxo-4H-imidazo [1,5-a] [1,4] benzodiazépine-3-carboxylate d'éthyle,
- 9,10-difluoro-5,6-dihydro-5-méthyl-6-oxo-4H-imidazo [1,5-a] [1,4] benzodiazépine-3-carboxylate d'éthyle,
- 5,6-dihydro-5-méthyl-6-oxo-7,8,9,10-tétrafluoro-4H-imidazo [1,5-a] [1,4] benzodiazépine-3-carboxylate d'éthyle,
- 5,6-dihydro-5-(4-méthoxyphényl)-6-oxo-4H-imidazo [1,5-a] [1,4] benzodiazépine-3-carboxylate d'éthyle,
- 5,6-dihydro-5-(4-méthoxyphényl)-6-oxo-4H-imidazo [1,5-a] [1,4] benzodiazépine-3-yl-cyclopropylméthanone,
- 7-chloro-5,6-dihydro-5-méthyl-6-oxo-4H-imidazo [1,5-a] [1,4] benzodiazépine-3-yl-cyclopropylméthanone,
- 5,6-dihydro-5-méthyl-6-oxo-7-trifluorométhyl-4H-imidazo [1,5-a] [1,4] benzodiazépine-3-yl-cyclopropylméthanone,
- 5,6-dihydro-7-fluoro-5-méthyl-6-oxo-4H-imidazo [1,5-a] [1,4] benzodiazépine-3-yl-cyclopropylméthanone,
- 5,6-dihydro-6-oxo-4H-imidazo [1,5-a] [1,4] benzodiazépine-3-yl-cyclopropylméthanone,
- 7-chloro-3-diéthyloxyméthyl-5,6-dihydro-5-méthyl-4H-imidazo [1,5-a] [1,4] benzodiazépine-6-oxo (4H)-one,
- 5,6-dihydro-10-amino-5-méthyl-6-oxo-4H-imidazo [1,5-a] [1,4] benzodiazépine-3-carboxylate d'éthyle,
- 5,6-dihydro-10-éthoxyméthylèneamino-5-méthyl-6-oxo-4H-imidazo [1,5-a] [1,4] benzodiazépine-3-carboxylate d'éthyle,
- 5,6-dihydro-10-(diméthylamino-méthylèneamino)-5-méthyl-6-oxo-4H-imidazo [1,5-a] [1,4] benzodiazépine-3-carboxylate d'éthyle,
- 5,6-dihydro-5-méthyl-10-méthylamino-6-oxo-4H-imidazo [1,5-a] [1,4] benzodiazépine-3-carboxylate d'éthyle,
- 5,6-dihydro-5-méthyl-10-méthylthio-6-oxo-4H-imidazo [1,5-a] [1,4] benzodiazépine-3-carboxylate d'éthyle,
- 5,6-dihydro-5-méthyl-10-thiocyano-6-oxo-4H-imidazo [1,5-a] [1,4] benzodiazépine-3-carboxylate d'éthyle,
- 5,6-dihydro-10-isothiocyano-5-méthyl-6-oxo-4H-imidazo [1,5-a] [1,4] benzodiazépine-3-carboxylate d'éthyle,

- 5,6-dihydro-10-iodo-5-méthyl-6-oxo-4H-imidazo [1,5-a] [1,4] benzodiazépine-3-carboxylate d'éthyle,
- 5,6-dihydro-5-méthyl-10-thioacétyl-6-oxo-4H-imidazo [1,5-a] [1,4] benzodiazépine-3-carboxylate d'éthyle, ainsi que leurs sels d'addition avec les acides.

Parmi les produits de formule (I) que le procédé de l'invention permet d'obtenir, on peut également citer les produits (appelés de formule ($I_E$)) dont les noms suivent :

- 5,6-dihydro-5-méthyl-7-nitro-6-oxo-4H-imidazo [1,5-a] [1,4] benzodiazépine-3-carboxylate d'éthyle,
- 5,6-dihydro-5-méthyl-8-nitro-6-oxo-4H-imidazo [1,5-a] [1,4] benzodiazépine-3-carboxylate d'éthyle,
- 7-chloro-5,6-dihydro-5-méthyl-6-oxo-4H-imidazo [1,5-a] [1,4] benzodiazépine-3-carboxylate d'éthyle,
- 5,6-dihydro-5-méthyl-6-oxo-7-trifluorométhyl-4H-imidazo [1,5-a] [1,4] benzodiazépine-3-carboxylate d'éthyle,
- 5,6-dihydro-7-iodo-5-méthyl-6-oxo-4H-imidazo [1,5-a] [1,4] benzodiazépine-3-carboxylate d'éthyle,
- 5,6-dihydro 7-fluoro 5-méthyl 6-oxo 4H-imidazo [1,5-a] [1,4] benzodiazépine-3-carboxylate d'éthyle, ainsi que leurs sels d'addition avec les acides.

Les produits de formule ($I_D$) peuvent être préparés selon les procédés décrits ci-dessus, comme indiqué dans les exemples ci-après.

Les produits de formule ($I_D$) possèdent de très intéressantes propriétés pharmacologiques, on note en particulier de faibles propriétés agonistes inverses au niveau des récepteurs des benzodiazépines. Tous les produits présentent par contre une plus longue durée d'action en comparaison d'autres imidazobenzodiazépines similaires. Certains produits présentent, en outre, des propriétés tranquilisantes. Ces propriétés sont illustrées plus loin dans la partie expérimentale.

Ces propriétés justifient l'utilisation des nouvelles imidazobenzodiazépines de formule ($I_D$) telles que définies ci-dessus, ainsi que leurs sels pharmaceutiquement acceptables, à titre de médicaments.

Parmi les médicaments préférés de l'invention, on retient tout particulièrement :

- 7-chloro-5,6-dihydro-5-méthyl-6-oxo-4H-imidazo [1,5-a] [1,4] benzodiazépine-3-yl-cyclopropylméthanone,
- 5,6-dihydro-5-méthyl-6-oxo-7-trifluorométhyl-4H-imidazo [1,5-a] [1,4] benzodiazépine-3-yl-cyclopropylméthanone,
- 5,6-dihydro-7-fluoro-5-méthyl-6-oxo-4H-imidazo [1,5-a] [1,4] benzodiazépine-3-yl-cyclopropylméthanone.

Ces médicaments trouvent, par exemple, leur emploi dans le traitement des troubles de la mémoire, notamment en gériatrie, des troubles de la sénescence cérébrale. Certains produits peuvent également être utilisés dans le traitement de l'obésité ainsi que comme tranquilisant mineur dans le traitement de certaines agitations ou irritabilité et dans certaines formes d'épilepsie.

La dose usuelle variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule ($I_D$) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a enfin pour objet, à titre de produits nouveaux et notamment d'intermédiaires nécessaires à la préparation des produits de formule (I) :

- les produits de formule (IV) :

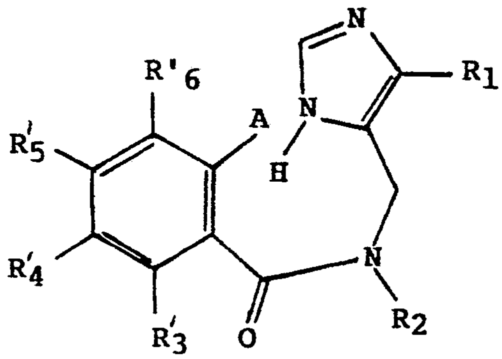

(IV)

dans laquelle $R_1$, $R_2$, $R'_3$, $R'_4$, $R'_5$, $R'_6$ et A ont la signification déjà indiquée.

Certains produits de formule (V) et (VII) sont décrits dans la demande de brevet européen n° 0 305 298.

- les produits de formule (V) :

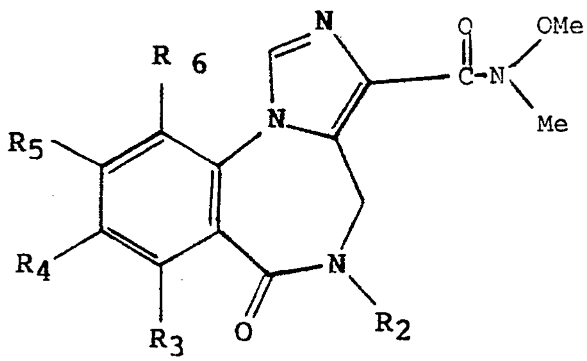

(V)

dans laquelle $R_2$, $R_3$, $R_4$, $R_5$ et $R_6$ ont la signification déjà indiquée à l'exception des produits de formule (V') :

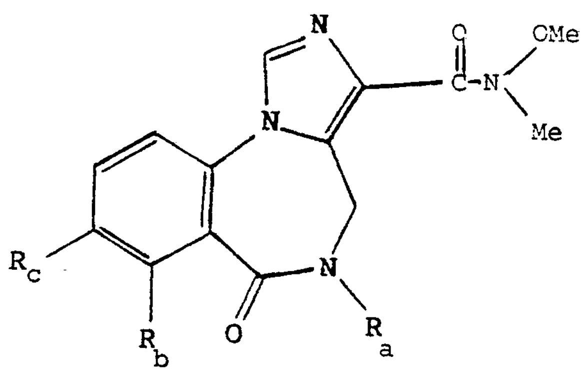

(V')

dans laquelle $R_b$ et $R_c$, identiques ou différents, représentent un atome d'hydrogène, un atome d'halogène, un groupe nitro, nitrile ou trifluorométhyle, $R_a$ représente un atome d'hydrogène, un radical alkyle renfermant de 1 à 5 atomes de carbone ou un groupe cycloalkyle renfermant de 3 à 5 atomes de carbone.

- Les produits de formule (VII) :

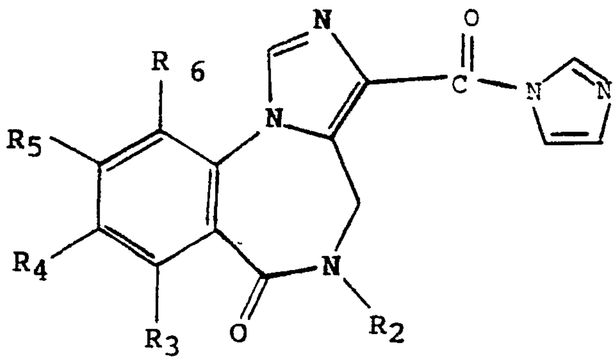

(VII)

dans laquelle $R_2$, $R_3$, $R_4$, $R_5$ et $R_6$ ont la signification déjà indiquée, à l'exception des produits de formule (VII') :

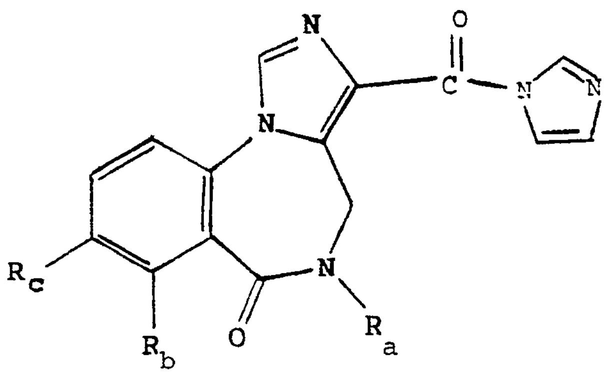

(VII')

dans laquelle $R_a$, $R_b$ et $R_c$ ont la signification déjà indiquée.

Les produits de formule (III) lorsqu'ils ne sont pas connus peuvent être préparés en utilisant le schéma réactionnel suivant :

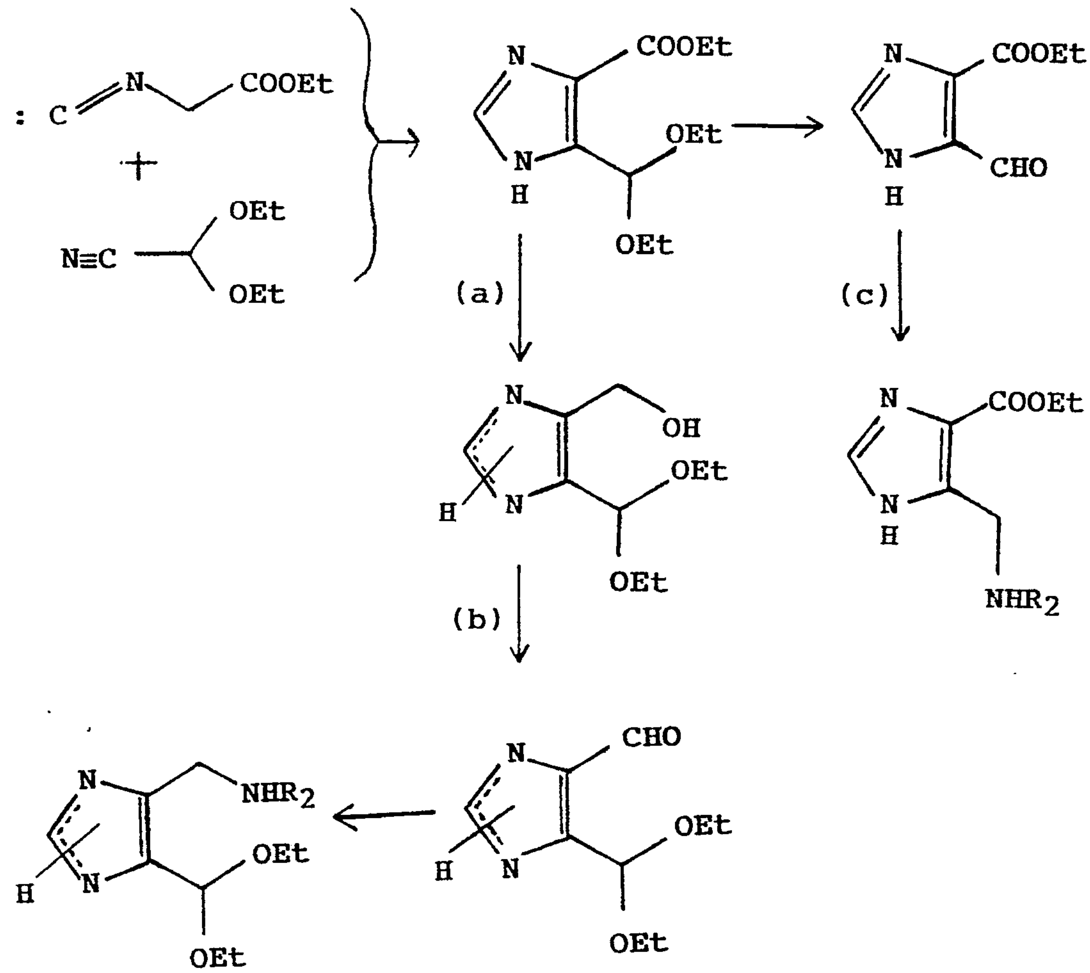


Les exemples suivants illustrent l'invention sans toutefois la limiter.

**PREPARATIONS.**

a) Préparation du 5(4)-diéthoxyméthyl-4(5)-hydroxyméthyl-1H-imidazole.

A une solution de 5(4)-diéthoxyméthyl-1H-imidazole 4(5)-carboxylate d'éthyle (préparée par le procédé de T. Murakami, M. Oksuka, M. Ohno, Tetrahedron Letters 1982 23 (45) 4729-4732) (10 g) dans du tétrahydrofuranne anhydre (500 ml), on ajoute sous agitation à 5° C sous atmosphère inerte en l'espace d'une heure, de l'aluminohydrure de lithium (4 g) et on agite le mélange pendant 1 heure. On ajoute goutte à goutte une solution aqueuse saturée de chlorure de sodium (50 ml) et on agite le mélange pendant 1/2 h, décante, lave avec du dichlorométhane (2 x 200 ml) et on sèche les extraits organiques sur $MgSO_4$ et évapore pour obtenir le 5(4)-diéthoxyméthyl-4(5)-hydroxyméthyl-1H-imidazole (8,14 g, 98 %) sous forme d'une huile.

b) Préparation du 5(4)-diéthoxyméthyl-1H-imidazole-4(5)-carboxaldéhyde.

A une solution de 5(4)-diéthoxyméthyl-4(5)-hydroxyméthyl-1H-imidazole (6,6 g) dans le dichlorométhane (250 ml), on ajoute du dioxide de manganèse activé (33 g), porte le mélange au reflux pendant 1/2 h puis filtre sur célite. L'évaporation du solvant donne le produit attendu (5,21 g, 84 %).

c) Préparation du 5(4)-méthylaminométhyl-1H-imidazole-4(5)-carboxylate d'éthyle.

**Procédé 1 (cyanoborohydrure de sodium)**

A une solution de 5-formyl-1H-imidazole-4-carboxylate d'éthyle (préparé selon le procédé de T. Murakami et al) (17 g) dans l'éthanol anhydre (170 ml) contenant des tamis moléculaires 3A (25 g), on ajoute une solution de méthylamine (33 % en poids/volume dans l'éthanol) (58 ml), puis du cyanoborohydrure de sodium (4,25 g). A ce mélange, on ajoute, tout en refroidissant (< 25° C), du chlorure d'hydrogène 2N dans l'éthanol jusqu'à ce que le mélange soit juste acide, au vert de bromocrésol (pH ≈ 4) et on agite le mélange pendant 24 h en maintenant le pH en ajoutant, si nécessaire HCl/éthanol.

On stoppe la réaction par addition successive de HCl 2N dans l'éthanol (100 ml) et d'eau (100 ml), on alcalinise le mélange réactionnel avec une solution aqueuse saturée de carbonate de potassium carbonate et on extrait avec du dichlorométhane. La solution organique est lavée avec de l'eau, séchée sur $MgSO_4$ et évaporée pour donner du 5(4)-méthylaminométhyl-1H-imidazole-4(5)-carboxylate d'éthyle (18,1 g) (70 % pureté 70% par CLHP).

**Procédé 2 (réduction catalytique)**

A une solution de 5-formyl-1H-imidazole-4-carboxylate d'éthyle (5,05 g) dans 200 ml d'éthanol, on ajoute une solution de méthylamine (33 % en poids/volume dans l'éthanol) (9,4 ml) et on agite le mélange pendant 30 min. On ajoute du palladium à 5% sur charbon actif (900 mg) et on soumet le mélange à une hydrogénation sous 0,4 bar. La filtration du mélange et l'évaporation du solvant donne du 5(4)-méthylaminométhyl-1H-imidazole-4(5)-carboxylate (5,5 g, 98 %) F = 105-107° C.

Par un procédé analogue, en utilisant les produits de départ appropriés, on prépare :

- le 5(4)-(4-méthoxyanilino)-méthyl-1H-imidazole-4(5)-carboxylate d'éthyle,
- le 5(4)-diéthoxyméthyl-4(5)-méthylaminométhyl-1H-imidazole sous forme d'huile.

Des exemples de l'utilisation de ces imidazoles dans la préparation des imidazobenzodiazépines sont indiqués ci-dessous.

**EXEMPLE 1 : 5,6-dihydro-5-méthyl-10-nitro-6-oxo-4H-imidazo [1,5-a] [1,4] benzodiazépine-3-carboxylate d'éthyle.**

Stade A : 5(4)-N-(2-chloro-3-nitrobenzoyl) méthylamino méthyl-1H-imidazole-4(5)-carboxylate d'éthyle.

A une suspension de 5(4)-methylaminomethyl-1H-imidazole-4(5)-carboxylate d'éthyle (1,3 g) dans le dichloromethane (50 ml), on ajoute du carbonate de sodium (1,5 g) et du chlorure de 2-chloro 3-nitrobenzoyle et on agite le mélange pendant 18 heures à la température ambiante. On filtre, chromatographie sur colonne ($SiO_2$ 0-5 % méthanol dans le dichlorométhane) et obtient le 5(4)-N-(2-chloro-3-nitrobenzoyl) méthylaminométhyl-1H-imidazole 4(5)-carboxylate d'éthyle (1,29 g, 53 %) sous forme d'une huile.

En opérant de manière analogue, on a préparé les composés suivants :

- 5(4)-N-(2-chloro-5-nitrobenzoyl) méthylaminométhyl-1H-imidazole-4(5)-carboxylate d'éthyle,
- 5(4)-N-(2,3,6-trifluorobenzoyl) méthylaminométhyl-1H-imidazole-4(5)-carboxylate d'éthyle,
- 5(4)-N-(2,3-difluorobenzoyl) méthylaminométhyl-1H-imidazole-4(5)-carboxylate d'éthyle,
- 5(4)-N-(2,3,4-trifluorobenzoyl) méthylaminométhyl-1H-imidazole-4(5)-carboxylate d'éthyle,
- 5(4)-N-(2,3,4,5,6-pentafluorobenzoyl) éthylaminométhyl-1H-imidazole-4(5)-carboxylate d'éthyle,
- 5(4)-N-(2-chloro-6-nitrobenzoyl) méthylaminométhyl-1H-imidazole-4(5)-carboxylate d'éthyle,
- 5(4)-N-(2-fluoro-6-chlorobenzoyl) méthylaminométhyl-1H-imidazole-4(5)-carboxylate d'éthyle,
- 5(4)-N-(2-fluoro-6-trifluorométhylbenzoyl) méthylaminométhyl-1H-imidazole-4(5)-carboxylate d'éthyle,
- 5(4)-N-(2-fluoro-6-iodobenzoyl) méthylaminométhyl-1H-imidazole-4(5)-carboxylate d'éthyle,
- 5(4)-N-(2,6-difluorobenzoyl) méthylaminométhyl-1H-imidazole-4(5)-carboxylate d'éthyle.

Stade B : 5,6-dihydro-5-méthyl-10-nitro-6-oxo-4H-imidazo [1, 5-a] [1,4] benzodiazépine-3-carboxylate d'éthyle.

A une solution de 5(4)-N-(2-chloro-3-nitrobenzoyl) méthylaminométhyl-1H-imidazole 4(5)-carboxylate d'éthyle (200 mg) dans le diméthylformamide (2 ml), on ajoute de l'hydrure de lithium (6 mg) et on chauffe le mélange à 100° C pendant 1 h. Le mélange est refroidi et l'excès d'hydrure de lithium neutralisé par l'addition d'un peu d'acide acétique. On dilue avec de l'acétate d'éthyle, lave avec de l'eau, sèche avec du sulfate de magnésium, et obtient après évaporation du solvant, une huile qui, par chromatographie sur silice ($CH_2Cl_2$ + $Et_2O$ de 0 % à 25 %) donne le 5,6-dihydro-5-methyl-10-nitro-6-oxo-4H-imidazo [1,5-a] [1,4] benzodiazepine-3-carboxylate d'éthyle (154 mg, 85 %). F = 136-137° C (dans l'éther).

En opérant de manière analogue, on a préparé les composés suivants :

**EXEMPLE 1B : 5,6-dihydro-5-méthyl-7-nitro-6-oxo-4H-imidazo [1,5-a] [1,4] benzodinzépine-3-carboxylate d'éthyle,**

**EXEMPLE 1C : 5,6-dihydro-5-méthyl-8-nitro-6-oxo-4H-imidazo [1,5-a] [1,4] benzodiazépine-3-car-**

**boxylate d'éthyle,**

**EXEMPLE 1D : 5,6-dihydro-10-fluoro-5-méthyl-6-oxo-4H-imidazo [1,5-a] [1,4] benzodiazépine-3-carboxylate d'éthyle,**

**EXEMPLE 1E : 7,10-difluoro-5,6-dihydro-5-méthyl-6-oxo-4H-imidazo [1,5-a] [1,4] benzodiazépine-3-carboxylate d'éthyle,**

**EXEMPLE 1F : 9,10-difluoro-5,6-dihydro-5-méthyl-6-oxo-4H-imidazo [1,5-a] [1,4] benzodiazépine-3-carboxylate d'éthyle,**

**EXEMPLE 1G : 5,6-dihydro-5-méthyl-6-oxo-7,8,9,10-tétrafluoro-4H-imidazo [1,5-a] [1,4] benzodiazépine-3-carboxylate d'éthyle,**

**EXEMPLE 1H : 7-chloro-5,6-dihydro-5-méthyl-6-oxo-4H-imidazo [1,5-a] [1,4] benzodiazépine-3-carboxylate d'éthyle,**

**EXEMPLE 1I : 5,6-dihydro-5-méthyl-6-oxo-7-trifluorométhyl-4H-imidazo [1,5-a] [1,4] benzodiazépine-3-carboxylate d'éthyle,**

**EXEMPLE 1J : 5,6-dihydro-7-iodo-5-méthyl-6-oxo-4H-imidazo [1,5-a] [1,4] benzodiazépine-3-carboxylate d'éthyle,**

**EXEMPLE 1K : 5,6-dihydro-7-fluoro-5-méthyl-6-oxo-4H-imidazo [1,5-a] [1,4] benzodiazépine-3-carboxylate d'éthyle,**

**EXEMPLE 2 : 5,6-dihydro-5-(4-méthoxyphényl)-6-oxo-4H-imidazo [1,5-a] [1,4] benzodiazépine-3-carboxylate d'éthyle.**

Stade A : 5,6-dihydro-10-amino-5-(4-méthoxyphényl)-6-oxo-4H-imidazo [1,5-a] [1,4] benzodiazépine-3-carboxylate d'éthyle.

On porte au reflux du 5,6-dihydro-5-(4-méthoxyphényl)-10-nitro-6-oxo-4H-imidazo [1,5-a] [1,4] benzodiazépine-3-carboxylate d'éthyle (préparé comme dans l'exemple 1) (820 mg, 9,94 mmol) dans un mélange de méthanol (30 ml) - dioxane (10 ml) avec du palladium à 10% sur charbon actif (100 mg) et du formiate d'ammonium (400 mg, 6,4 mmol) pendant 1 h. Après filtration sur célite et évaporation du solvant on obtient une conversion quantitative en 5,6-dihydro-10-amino-5-(4-méthoxyphényl)-6-oxo-4H-imidazo [1,5-a] [1,4] benzodiazépine-3-carboxylate d'éthyle. Cette substance est utilisée dans l'étape suivante sans autre purification.

Stade B : 5,6-dihydro-5-(4-méthoxyphényl)-6-oxo-4H-imidazo [1,5-a] [1,4] benzodiazépine-3-carboxylate d'éthyle.

On porte 3 heures au reflux le 5,6-dihydro-10-amino-5-(4-méthoxyphényl)-6-oxo-4H-imidazo [1,5-a] [1,4] benzodiazépine-3-carboxylate d'éthyle préparé au stade A ci-dessus dans de l'éthanol anhydre (50 ml) en présence de nitrite d'isoamyle (1,8 ml, 13,4 mmol), et obtient après élimination du solvant et purification sur gel de silice, le 5,6-dihydro-5-(4-méthoxyphényl)-6-oxo-4H-imidazo [1,5-a] [1,4] benzodiazépine-3-carboxylate d'éthyle (429 mg, 58,7 % pour les 2 étapes).

**EXEMPLE 3 : 5,6-dihydro-5-(4-méthoxyphényl)-6-oxo-4H-imidazo [1,5-a] [1,4] benzodiazépine-3-yl-cyclopropylméthanone.**

Stade A : (5,6-dihydro-5-(4-méthoxyphényl)-6-oxo-4H-imidazo [1,5-a] [1,4] benzodiazépine-3-yl) N-méthyl carbohydroxamate de méthyle.

On dissout dans de l'acétone (5 ml) le 5,6-dihydro-5-(4-méthoxyphényl)-6-oxo-4H-imidazo [1,5-a] [1,4] benzodiazépine-3-carboxylate d'éthyle (396 mg, 1,05 mmoles) obtenu au stade B de l'exemple 2 et on agite avec une solution aqueuse de potasse (176 mg, 2,63 mmol dans 1,6 ml $H_2O$) pendant 2 heures à la température ambiante. On l'abandonne pendant 18 heures à 4° C, acidifie la solution à pH 3-4 avec HCl concentré, puis filtre. Le solide récupéré est lavé avec de l'acétate d'éthyle pour éliminer la substance de départ (149 mg) puis séché et agité dans le chlorure de méthylène (7 ml) en présence de 1,1-carbonyldiimidazole (178 mg, 1,1 mmoles) pendant 4 heures. Une quantité supplémentaire de réactif (64 mg, 0,4 mmole) est ajoutée et la solution est agitée pendant encore 2 heures.

L'addition de chlorhydrate N,O-diméthylhydroxylamine (195 mg, 2,0 mmoles) suivie d'une quantité supplémentaire de (49 mg, 0,5 mmoles) au bout de 6 heures, et une agitation pendant 16 heures donnent l'ester hydroxamate. Après filtration et chromatographie, on obtient le (5,6-dihydro-5-(4-méthoxyphényl)-6-oxo-4H-imidazo [1,5-a] [1,4] benzodiazépine-3-yl) N-méthyl carbohydroxamate de méthyle (236 mg, 57 % par rapport à l'ester).

Stade B : 5,6-dihydro-5-(4-méthoxyphényl)-6-oxo-4H-imidazo [1,5-a] [1,4] benzodiazépine-3-yl-cyclopropylméthanone.

On traite l'ester hydroxamate (215 mg, 0,54 mmole) dans le THF anhydre (5 ml) à -78 °C avec une solution fraîchement préparée de bromure de cyclopropylmagnésium dans le THF (2,2 mmoles dans 3,5 ml). Après avoir réchauffé à la température ambiante, on ajoute de l'acide acétique (0,5 ml), dilue avec $CH_2Cl_2$, lave avec de l'eau, sèche et évapore. Après chromatographie sur gel de silice ($CH_2Cl_2$ : ether 9-1) et cristallisation dans l'éther, on obtient le 5,6-dihydro-5-(4-méthoxyphényl)-6-oxo-4H-imidazo [1,5-a] [1,4] benzodiazépine-3-yl-cyclopropylméthanone (72 mg, 36 %).

En utilisant un procédé analogue à celui décrit dans les étapes (a) et (b) à partir des esters éthyliques appropriés, on a préparé les produits suivants :

**EXEMPLE 3B : 7-chloro-5,6-dihydro-5-méthyl-6-oxo-4H-imidazo [1,5-a] [1,4] benzodiazépine-3-yl-cyclopropylméthanone,**

**EXEMPLE 3C : 5,6-dihydro-5-méthyl-6-oxo-7-trifluorométhyl-4H-imidazo [1,5-a] [1,4] benzodiazépine-3-yl-cyclopropylméthanone,**

**EXEMPLE 3D : 5,6-dihydro-7-fluoro-5-méthyl-6-oxo-4H-imidazo [1,5-a] [1,4] benzodiazépine-3-yl-cyclopropylméthanone.**

**EXEMPLE 4 : 5,6-dihydro-6-oxo-4H-imidazo [1,5-a] [1,4] benzodiazépine-3-yl-cyclopropylméthanone.**

On agite de la 5,6-dihydro-5-(4-méthoxyphényl)-6-oxo-4H-imidazo [1,5-a] [1,4] benzodiazépine-3-yl-cyclopropylméthanone (15 mg, 0,04 mmoles) préparé au stade B de l'exemple 3, dans $CH_3CN$ avec du nitrate de cérium (IV) ammonium (66 mg, 0,12 mmoles) dans l'eau (0,3 ml). Au bout d'une heure, on ajoute une quantité supplémentaire de 44 mg (0,08 mmole) dans de l'eau (0,2 ml) et on agite la solution pendant une heure supplémentaire. L'évaporation, l'extraction avec $CH_2Cl_2$ et la trituration du solide récupéré avec de l'éther donnent le composé attendu (8 mg, 74 %).

**EXEMPLE 5 : 7-chloro-3-diethoxyméthyl-5,6-dihydro-5-méthyl-4H-imidazo [1,5-a] [1,4] benzodiazépine-6(4H)-one.**

Stade A : 5(4)-N-(2-chloro-6-fluorobenzoyl) méthylamino-4(5)-diéthoxyméthyl-1H-imidazole.

A une suspension d'acide 2-chloro-6-fluorobenzoïque (1,74 g) dans le dichlorométhane (30 ml), on ajoute du N,N-dicyclohexylcarbodiimide (2,06 g) et du 1-hydroxybenzotriazole (1,35 g) et on agite le mélange à la température ambiante pendant 1 heure avant d'éliminer la dicyclohexylurée par filtration et obtient une solution de l'ester actif.

A une solution de 5(4)-diéthoxyméthyl-4(5)-méthylaminométhyl-1H-imidazole (2,18 g), on ajoute en une heure, la moitié de la solution d'ester actif préparée ci-dessus. On agite le mélange à la température ambiante pendant 3 heures, puis on ajoute un quart supplémentaire de la solution d'ester actif. On agite 2 heures puis lave la solution organique avec une solution aqueuse de carbonate de potassium et on l'évapore pour obtenir une huile.Après chromatographie sur colonne ($SiO_2$, 0-5 % éthanol dans le dichlorométhane), on obtient le 5(4)-N-(2-chloro-6-fluorobenzoyl) méthylamino-4(5)-diéthoxy méthyl-1H-imidazole (2,17 g, 57 %).

Stade B : 7-chloro-3-diéthoxyméthyl-5,6-dihydro-5-méthyl-4H-imidazo [1,5-a] [1,4] benzodiazépine-6(4H)-one.

A une solution de 5(4)-N-(2-chloro-6-fluorobenzoyl) méthylamino-4(5)-diéthoxyméthyl-1H-imidazole (2 g) dans le diméthylformamide anhydre (40 ml), on ajoute de l'hydrure de lithium (175 mg) et soumet le mélange à des ultrasons pendant 15 minutes, puis le chauffe à 100 °C pendant 3 heures. Après l'avoir refroidi et avoir éliminé l'excès d'hydrure de lithium par filtration, on dilue la solution avec de l'acétate d'éthyle (200 ml), on la lave avec de l'eau et on l'évapore pour obtenir une huile.

Après chromatographie sur colonne ($SiO_2$ 1 % éthanol dans le dichlorométhane) et cristallisation dans un système éther/pétrole, on obtient la 7-chloro-3-diéthoxyméthyl-5,6-dihydro-5-méthyl-4H-imidazo [1,5-a] [1,4] benzodiazépine-6(4H)-one (1,16 g, 61 %).

**EXEMPLE 6 : 5,6-dihydro-10-amino-5-méthyl-6-oxo-4H-imidazo [1,5-a] [1,4] benzodiazépine-3-carboxylate d'éthyle.**

On réduit le 5,6-dihydro-5-méthyl-10-nitro-6-oxo-4H-imidazo [1,5-a] [1,4] benzodiazépine-3-carboxylate

d'éthyle (5,01 g, 15 mmoles) (préparé comme dans l'exemple 1) en 5,6-dihydro-10-amino-5-méthyl-6-oxo-4H-imidazo [1,5-a] [1,4] benzodiazépine-3-carboxylate d'éthyle par un procédé analogue à celui décrit au stade A de l'exemple 2, pour obtenir un solide cristallin dans un sustème méthanol/chloroforme (4,3 g, 96 %).

**EXEMPLE 7A : 5,6-dihydro-10-éthoxyméthylèneamino-5-méthyl-6-oxo-4H-imidazo [1,5-a] [1,4] benzodiazépine-3-carboxylate d'éthyle.**

On porte au reflux sous atmosphère inerte, pendant 5 jours, du 5, 6-dihydro-10-amino-5-méthyl-6-oxo-4H-imidazo [1,5-a] [1,4] benzodiazépine-3-carboxylate d'éthyle (1,2 g, 4 mmoles) (préparé comme dans l'exemple 6) dans de l'orthoformiate de triéthyle non dilué. La solution est ensuite évaporée et l'huile obtenue est chromatographié ($CH_2Cl_2$ : acétone 9-1) pour donner le dérivé 10-éthoxyméthylèneamino (1,05 g, 73,7 %).

**EXEMPLE 7B : 5,6-dihydro-10-méthylamino-méthylèneamino-5-méthyl-6-oxo-4H-imidazo [1,5-a] [1,4] benzodiazepine-3-carboxylate d'éthyle.**

En utilisant le diméthylformamide-diéthylacétal et en procédant de manière analogue à l'exemple 7A, on a obtenu le produit attendu avec un rendement de 46,4%

**EXEMPLE 8 : 5,6-dihydro-5-méthyl-10-méthylamino-6-oxo-4H-imidazo [1, 5-a] [1,4] benzodiazépine-3-carboxylate d'éthyle.**

On agite pendant 16 heures le 5,6-dihydro-10-éthoxyméthylèneamino-5-méthyl-6-oxo-4H-imidazo [1,5-a] [1,4] benzodiazépine-3-carboxylate d'éthyle (préparé comme à l'exemple 7A) (964 mg, 2,71 mmoles) dans l'éthanol (10 ml) avec du borohydrure de sodium (150 mg, 3,95 mmol). On ajoute de nouveau du borohydrure de sodium (150 mg) et agite la solution pendant 24 heures. Le produit cristallin est filtré et lavé avec de l'éthanol, pour donner le dérivé 10-méthylamino recherché (654 mg, 78,5 %).

**EXEMPLE 9A : 5,6-dihydro-5-méthyl-10-méthylthio-6-oxo-4H-imidazo [1,5-a] [1,4] benzodiazépine-3-carboxylate d'éthyle.**

On traite le 5, 6-dihydro-10-amino-5-méthyl-6-oxo-4H-imidazo [1,5-a] [1,4] benzodiazépine-3-carboxylate d'éthyle (préparé comme à l'exemple 6) dans un système acide trifluoroacétique (2 ml)/méthanol/eau à 0° C avec du nitrite de butyle à 90 % (48 ml, 0,36 mmole). Après avoir agité à 0-5° C pendant 10 minutes, on ajoute lentement une solution aqueuse de MeSCu (100 mg, 0,99 mmole) et on agite le mélange pendant 15 minutes à 0° C. Après évaporation à sec et addition d'une solution aqueuse de bicarbonate de sodium, on extrait avec du chloroforme, sèche sur $MgSO_4$ et élimine le solvant et obtient une gomme, qui après chromatographie, donne le produit recherché (58 mg, 53 %).

**EXEMPLES 9B ET 9C : 5,6-dihydro-5-méthyl-10-thiocyano-6-oxo-4H-imidazo [1,5-a] [1,4] benzodiazépine-3-carboxylate d'éthyle et 5,6-dihydro-10-isothiocyano-5-méthyl-6-oxo-4H-imidazo [1,5-a] [1,4] benzodiazepine-3-carboxylate d'éthyle.**

En opérant de manière analogue en utilisant du thiocyanate de cuivre (I), on a préparé les produits sous forme de mélange et la séparation par chromatographie donne respective ment 15,8 % et 19,4 % des produits thiocyano et isothiocyano.

**EXEMPLE 10 : 5,6-dihydro-10-iodo-5-méthyl-6-oxo-4H-imidazo [1,5-a] [1,4] benzodiazépine-3-carboxylate d'éthyle.**

On refroidit à -78° C sous atmosphère inerte le 5,6-dihydro-10-amino-5-méthyl-6-oxo-4H-imidazo [1,5-a] [1,4] benzodiazépine-3-carboxylate d'éthyle (préparé comme dans l'exemple 6) (150 mg, 0,5 mmole) dans le THF anhydre (1 ml), et on ajoute goutte à goutte du trifluoroéthérate de bore (0,25 ml, 2 mmole) puis on ajoute goutte à goutte à -10° C, du nitrite de butyle (0,12 ml, 1 mmole). Après avoir agité pendant 30 minutes à cette température, on ajoute de l'éther, filtre la solution et sèche le solide sous pression réduite. Le sel de diazonium est ajouté lentement à l'iodure de sodium (225 mg, 1,5 mmol) dans l'acétone (6 ml) en 5 minutes. On agite une heure et demie à température ambiante puis la solution est évaporée et

chromatographiée pour donner, après cristallisation ($CH_2Cl_2$/ether) le produit attendu (92 mg, 44,9 %).

**EXEMPLE 10B : 5,6-dihydro-5-méthyl-10-thioacétyl-6-oxo-4H-imidazo [1,5-a] [1,4] benzodiazépine-3-carboxylate d'éthyle.**

En utilisant du thioacétate de potassium dans le THF, on a obtenu le produit attendu.

Les analyses spectrométriques, les rendements, les points de fusion et les résultats de la microanalyse sont donnés dans les tableaux ci-après.

TABLEAU I

| | $R_1$ | $R_2$ | $R_3$ | $R_4$ | $R_5$ | $R_6$ | Rendement | Pt de F °C · | I.r. $cm^{-1}$ |
|---|---|---|---|---|---|---|---|---|---|
| EXEMPLE 1 | COOEt | Me | H | H | H | $NO_2$ | 85% | 136-7 $Et_2O$ | 1698, 1643, 1536, 1347, 1262, 1184, 1073 |
| EXEMPLE 1G | COOEt | Me | F | F | F | F | 59% | 192-4 $CH_2Cl_2/Et_2O$ | 1738, 1660, 1502, 1225, 1213, 1157, 1058 |
| EXEMPLE 6 | COOEt | Me | H | H | H | $NH_2$ | | 314-16 $CHCl_3/MeOH$ | 3420, 3330, 3215, 1700, 1626, 1584, 1435 1373, 1153, 1030, 753 |
| EXEMPLE 8 | COOEt | Me | H | H | H | $NHCH_3$ | 78.5% | 238-240 | 3285, 1725, 1697, 1635, 1580, 1490, 1375 1150, 1140, 1177, 1067, 750 |
| EXEMPLE 9 | COOEt | Me | H | H | H | $SCH_3$ | 53% | 141-143 $CH_2Cl_2$:ether | 1684, 1634, 1487, 1367, 1255, 1193, 1075 750 |
| EXEMPLE 10 | COOEt | Me | H | H | H | I | 45% | $CH_2Cl_2$:ether 180-181 | 3100, 1695, 1634, 1625, 1520, 1485, 1385 1377, 1255, 1130, 1180, 1105, 1058, 1150 |
| EXEMPLE 7B | COOEt | Me | H | H | H | -N^NMe$_2$ | 46% | 218-230 EtOAc/EtOH | 2920, 1710, 1630, 1577, 1450, 1382, 1750 1190, 1127, 1080, 750 |
| EXEMPLE 1E | COOEt | Me | F | H | H | F | 53% | EtOAc/$Et_2O$ | 1692, 1650, 1640, 1495, 1340, 1213, 1192, 1077 |
| EXEMPLE 1F | COOEt | Me | H | H | F | F | 67% | 222-224 EtOAc/$Et_2O$ | 1726, 1638, 1618, 1510, 1482, 1394, 1196 1169, 1102 |
| EXEMPLE 1D | COOEt | Me | H | H | H | F | 25% | 184-186 $Et_2O$ | 1696, 1636, 1573, 1497, 1372, 1260, 1182 1075 |

TABLEAU I (suite)

| | $R_1$ | $R_2$ | $R_3$ | $R_4$ | $R_5$ | $R_6$ | Rendement | Pt de F °C | IR $cm^{-1}$ |
|---|---|---|---|---|---|---|---|---|---|
| EXEMPLE 2 | COOEt | 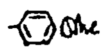 | H | H | H | H | 58.7% | 181-182 | 1723,1634,1581,1290,1248,1220,1168 1145,758 |
| EXEMPLE 3 | 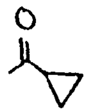 | 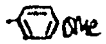 | H | H | H | H | 20.5% | 184-186 $Et_2O$ | 1160,1646,1511,1495,1387,1248,1220 1180,1044,965,948,990,768 |
| EXEMPLE 4 | 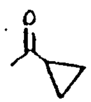 | H | H | H | H | H | 74% | 166-167 $Et_2O$ | 3180,1668,1640,1560,1495,1489,1390 1334,1248,1235,1020,995,958,928, 784,759 |
| EXEMPLE 3B | 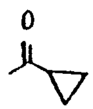 | Me | Cl | H | H | H | 75% | 190-192 | 1650,1562,1487,1380,1228,1029,1017 970,798 |
| EXEMPLE 3C | 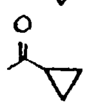 | Me | $CF_3$ | H | H | H | 65% | 222-224 $CH_2Cl_2/Et_2O$ | 1650,1566,1490,1383,1320,1305,1159 1137,1011,961 |
| EXEMPLE 3D | 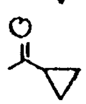 | Me | F | H | H | H | 68% | 198-200 $CH_2Cl_2/Et_2O$ | 1637,1564,1490,1385,1225,974,868, 803 |
| EXEMPLE 5 | $-CH(OEt)_2$ | -Me | Cl | H | H | H | 61% | 110-112 | 1635,1578,1476,1380,1100,1083,1046 1008,795 |

TABLEAU II

| | RMN de $^{1}H$ | Formule : Masse moléculaire | Analyse : Calculé Trouve C | H | N | Autres |
|---|---|---|---|---|---|---|
| EXEMPLE 1 | $CDCl_3$ 8.27(1H,dd); 8.06(1H,dd); 6.98(1H,t); 6.52(1H,s); 5.27(1H,d); 4.45(3H,dq + d); 3.23(3H,s); 1.47(3H,t) | $C_{15}H_{14}N_4O_5$ 330.303 | 54.55<br>54.53 | 4.27<br>4.35 | 16.96<br>16.89 | -<br>- |
| EXEMPLE 16 | $CDCl_3$ 7.98 (1H,d); 5.24(1H,d); 4.42(3H,d+m); 3.17(3H,s); 1.46(3H,t) | $C_{15}H_{11}F_4N_3O_3$ 357.268 | 50.43<br>50.27 | 3.10<br>3.21 | 11.76<br>11.57 | 21.27<br>20.99 |
| EXEMPLE 6 | $CDCl_3$/ $CF_3CO_2D$ 9.46(4H,s); 7,3-8(4H,m); 5.22(1H,d); 4.5(3H, q+d); 3.78(3H,s); 1.43(3H,t) | $C_{15}H_{16}N_4O_3$ 300.33 | 59.99<br>59.60 | 5.37<br>5.45 | 18.66<br>18.53 | |
| EXEMPLE 8 | $CDCl_3$ 8.07(1H,s); 7.26-7.4(2H,m); 6,92(1Hd,d); 5.07 (1H,d); 4.43(2H,m); 4.33(1H,d); 3.18(3H,s); 2.85(3H,d); 1.44(3H,t) | $C_{16}H_{18}N_4O_3$ 314.35 | 61.14 | 5.77 | 17.87 | |
| EXEMPLE 9 | $CDCl_3$ 8.13 (1H,s); 7.76(1H,dd); 7.52(2H,m); 5.13(1H,d); 4.43(2H,m); 4.30(1H,d); 3.19(3H,s); 2.43(3H,s); 1.45(3H,t) | $C_{16}H_{17}N_3O_3S$ 331.40 | 57.99<br>57.44 | 5.17<br>5.20 | 12.68<br>12.68 | 9.67<br>9.51 |
| EXEMPLE 10 | $CDCl_3$ 8.24(1H,bs); 8.16(1H,dd); 7.95(1H,dd); 7.25(1H,t); 5.14(1H,d); 4.44(2H,m); 4.31(1H,d); 3.19(3H,s); 1.46(3H,t) | | | | | |
| EXEMPLE 7B | $CDCl_3$ 8.2(1H,s); 7.57(1H,s); 7.55(1H,dd); 7.27(1H,dd); 7.07(1H,dd); 5.12(1H,d); 4.4(2H,q); 4.4(1H,d); 3.2(3H,s); 3.05(3H,s); 1.35(3H,s); 1.45(3H,t) | $C_{18}H_{21}N_5O_3$ 355.400 | 60.83<br>60.53 | 5.96<br>6.01 | 19.71<br>19.51 | |
| EXEMPLE 1E | $CDCl_3$ 7.99(1H,d);7.4(1H,m);7.25(1H,m);5.23(1H,d);4.44(2H,m); 3.20(3H,s);1.46(3H,t) | $C_{15}H_{13}F_2N_3O_3$ 321.286 | 56.08<br>56.07 | 4.08<br>4.11 | 13.08<br>13.09 | 11.83<br>11.85 |
| EXEMPLE 1F | $CDCl_3$ 8.0(1H,d); 7.85(1H,m); 7.63(1H,q); 5.42(1H,d); 4.44(3H,mtd); 3.24(3H,s); 1.46(3H,t) | $C_{15}H_{13}F_2N_3O_3$ 321.286 | 56.08<br>55.69 | 4.08<br>4.14 | 11.83<br>11.66 | 13.08<br>12.95 |
| EXEMPLE 1D | $CDCl_3$ 7.98(1H,d);7.83(1H,dd);7.45(2H,m);5.23(1H,d);4.46(2H,m); 4.35(1H,d);3.25(3H,s);1.46(3H,t) | $C_{15}H_{14}FN_3O_3$ 303.295 | 59.40<br>59.09 | 4.65<br>4.73 | 13.85<br>13.77 | 6.26<br>6.25 |

TABLEAU II (suite)

| | RMN de $^1H$ | Formule Masse moléculaire | Analyse : Calcule / Trouvé C | H | N | Autres |
|---|---|---|---|---|---|---|
| EXEMPLE 2 | $CDCl_3$ 8.17(1H,dd); 7.95(1H,s); 7.54-7.74(2H,m); 7.47(1H,dd); 7.20(2H,d); 6.90(2H,d); 5.55(1H,d); 4.71(1H,d); 4.32(2H,m); 3.82(3H,s); 1.26(3H,t) | | | | | |
| EXEMPLE 3 | $CDCl_3$ 8.16(1H,dd); 7.97(1H,s); 7.53-7.73(2H,m); 7.48(1H,dd); 7.19(2H,d); 6.89(2H,d); 5.67(1H,dd); 4.66(1H,bd); 3.80(3H,s); 3.18(1H,m); 1.04-1.24(4H,m) | $C_{22}H_{19}N_3O_3$ 373.42 | 70.76<br>70.72 | 5.13<br>5.19 | 11.25<br>11.28 | |
| EXEMPLE 4 | $CDCl_3$ 8.21(1H,dd); 7.90(1H,s); 7.56-7.74(2H,m); 7.47(1H,dd); 6.77(1H,t); 4.2-5.2(2H,bh); 3.19(1H,m); 1.03-1.26(4H,m) | $C_{15}H_{13}N_3O_2$ 267.29 | 67.40<br>66.96 | 4.90<br>4.96 | 15.72<br>15.61 | |
| EXEMPLE 3B | $CDCl_3$ 7.91(1H,s);7.56(2H,m);7.33(1H,dd);5.27(1Hpl);4.33(1H,d); 3.17(4H,s+m);1.23(2H,m);1.10(2H,m) | $C_{16}H_{14}N_3ClO_2$ | 60.86 | 4.47 | 13.31 | Cl11.23 |
| EXEMPLE 3C | $CDCl_3$ 7.95(1H,s);7.88(1H,dd);7.74(1H,dt);7.61(1H,dd);5.33(1H,d); 4.33(1H,d);3.20(1H,m);3.16(3H,s);1.23m);1.11(2h,m) | $C_{17}H_{14}F_3N_3O_2$ +¼$H_2O$ | 57.70<br>57.87 | 4.13<br>4.18 | 11.88<br>11.84 | F16.11<br>18.88 |
| EXEMPLE 3D | $CDCl_3$ 7.91(1H,s);7.61(1H,m);7.26(3H,s+m);5.31(1H,d);3.36(1H,d); 3.19(4H,s+m);1.20(4H,m) | $C_{16}H_{14}N_3O_2F$ 299.285 | 64.21<br>64.18 | 4.72<br>4.78 | 14.04<br>14.07 | F 6.35<br>6.33 |
| EXEMPLE 5 | $CDCl_3$ 7.8(1H,s);7.5(2H,m);7.26(1H,m);5.65(1H,s);4.79(1H,d); 4.31(1H,d);3.68(4H,m);3.19(4H,m);3.19(3H,s);1.28(6H,q) | $C_{17}H_{20}ClN_3C_3$ 349.82 | 58.37<br>58.34 | 5.76<br>5.62 | 12.01<br>12.26 | Cl10.13<br>10.31 |

**EXEMPLE 11 :**

On a préparé des comprimés répondant à la formule suivante :

- composé de l'exemple 3B ..................... 20 mg
- excipient q.s.p. un comprimé terminé à ......150 mg

(détail de l'excipient : lactose, amidon, talc, stéarate de magnésium).

**EXEMPLE 12 :**

On a préparé des comprimés répondant à la formule suivante :

- composé de l'exemple 3D ..................... 20 mg
- excipient q.s.p. un comprimé terminé à ......150 mg

(détail de l'excipient : lactose, amidon, talc, stéarate de magnésium).

Activité pharmacologique

Les composés sont des agents qui interagissent avec les récepteurs de benzodiazépine dans le cerveau, certains d'entre eux pouvant être utiles pour traiter l'obésité et le disfonctionnement cognitif et certains d'entre eux pouvant être utiles comme tranquillisants mineurs.

La recherche de la fixation aux récepteurs de benzodiazépine (FRB) est réalisée par le procédé décrit dans GB 2 128 989.

La mesure de la fixation in vivo aux récepteurs de benzodiazépine est réalisée selon le procédé décrit par Goeders N.E. and Kuhar M J, Life Sciences (1985) 37 345.

**Tableau III**

| Composé de l'exemple | FRBnM | Fixation in vivo DE50 mg/pk ip |
|---|---|---|
| 1G | 7500 | 100 |
| 6 | 966 | - |
| 1E | 174 | 1,1 |
| 1D | 410 | 21 |
| 4 | 582 | - |
| 3B | 7,7 | 0,032 |
| 3C | 9,4 | 0,05 |
| 3D | 27 | 0,035 |
| 5 | 339 | 2,1 |

## Revendications

**1.** Procédé de préparation d'imidazobenzodiazépines répondant à la formule générale ($I_A$) :

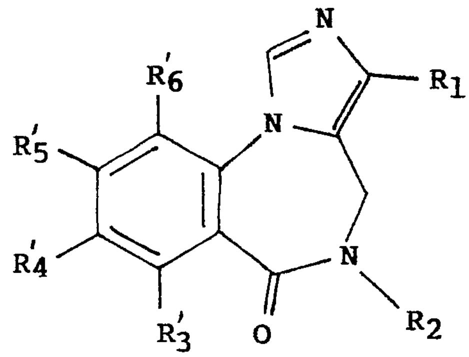

($I_A$)

dans laquelle :

$R_2$ représente un atome d'hydrogène, un radical alkyle renfermant de 1 à 5 atomes de carbone, un radical cycloalkyle renfermant de 3 à 6 atomes de carbone, un groupement phényle, un groupement phényle substitué,

$R'_3$, $R'_4$, identiques ou différents, représentent un atome d'hydrogène, un atome d'halogène, un groupement $CX_3$ dans lequel X représente un atome d'halogène, un groupement $NO_2$, un groupement CN, un groupement NRR', un groupement CONRR', dans lesquels R représente un atome d'hydrogène ou un radical alkyle renfermant de 1 à 5 atomes de carbone et R' représente un radical alkyle renfermant de 1 à 5 atomes de carbone, ou R et R' ensemble représentent un radical alkylène, renfermant de 2 à 5 atomes de carbone, soit $R'_3$ et $R'_4$ représentent un radical $OR_7$, $SR_7$, $COOR_7$ ou $COR_7$ dans lequel $R_7$ représente un atome d'hydrogène, un radical alkyle renfermant de 1 à 5 atomes de carbone ou un groupement phényl,

$R'_5$ représente un atome d'hydrogène ou de fluor et que :

- soit $R_1$ représente un groupement :

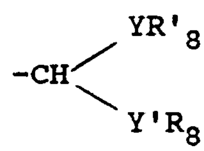

dans lequel $R_8$ et $R'_8$ identiques ou différents représentent un radical alkyle renfermant de 1 à 3 atomes de carbone ou $R_8$ et $R'_8$ ensemble représentent un groupe alkylène contenant de 2 à 5 atomes de carbone et Y et Y' identiques ou différents, représentent un atome d'oxygène ou un atome de soufre, et $R'_6$ représente un atome d'hydrogène, un atome d'halogène, un groupement nitro, un groupement amino, un groupement -NRR' dans lequel R et R' ont la signification déjà indiquée ou $R'_6$ représente un groupement $SR_7$, un groupement $OR_7$, un groupement $COR_7$, groupements dans lesquels $R_7$ a la signification déjà indiquée,

- soit $R_1$ représente un groupement $COOR_9$ dans lequel $R_9$ représente un radical alcoyle renfermant de 1 à 5 atomes de carbone ou $R_1$ représente un groupement cycloalkyle renfermant de 3 à 6 atomes de carbone, et $R'_6$ représente un atome d'hydrogène, un atome d'halogène, un groupement nitro, un groupement NRR', un groupement CONRR' dans lesquels R et R' ont la signification déjà indiquée, un radical $-OR_7$, $SR_7$, $COOR_7$ ou $COR_7$ dans lesquels $R_7$ a la signification déjà indiquée, ou un groupement $-N=CH-OR_9$ dans lequel $R_9$ a la signification déjà indiquée, ou $R'_6$ représente un groupement CN, un groupement amino, un groupement $-N=CH-N(CH_3)_2$ ou un groupement thiocyano, isothiocyano ou thioacétyle,

- soit $R_1$ représente un groupement $-COR'_c$ dans lequel $R'_c$ représente un groupement cycloalkyle renfermant de 3 à 6 atomes de carbone et $R'_6$ représente un atome d'hydrogène, un atome d'halogène, un groupement nitro, un groupement -NRR' ou -CONRR' dans lesquels R et R' ont la signification déjà indiquée, ou un groupement $-SR_7$, $-OR_7$, $-COOR_7$ ou $-COR_7$ dans lesquels $R_7$ a la signification déjà indiquée, ou un groupement $-N=CH-OR_9$ dans lequel $R_9$ a

la signification déjà indiquée, ou un groupement CN, à la condition que l'un au moins de $R'_3$, $R'_4$, $R'_5$ et $R'_6$ soit un groupe éléctro attracteur, ainsi que leurs sels, procédé caractérisé en ce que l'on fait réagir un produit de formule (II) :

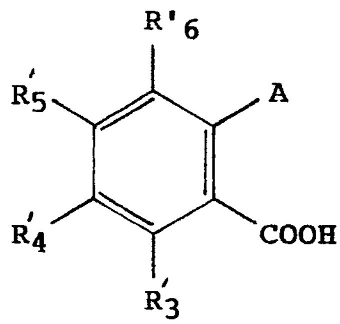

(II)

dans laquelle $R'_3$, $R'_4$, $R'_5$ et $R'_6$ ont la signification déjà indiquée, A représente un groupement éliminable ou un dérivé fonctionnel de cet acide de formule (II) avec un produit de formule (III) :

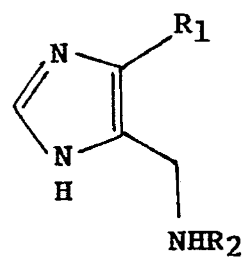

(III)

dans laquelle $R_1$ et $R_2$ ont la signification déjà indiquée, pour obtenir un produit de formule (IV) :

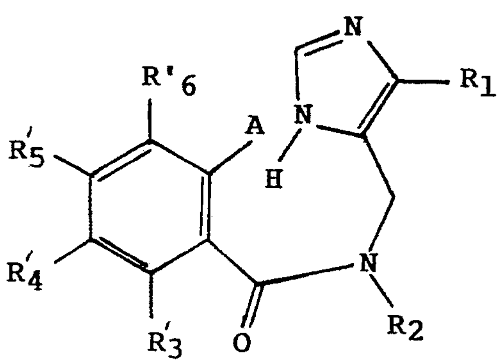

(IV)

dans laquelle $R_1$, $R_2$, $R'_3$, $R'_4$, $R'_5$, $R'_6$ et A ont la signification déjà indiquée, puis cyclise ce dernier et si désiré, salifie le produit de formule ($I_A$) obtenu.

**2.** Procédé de préparation selon la revendication 1, caractérisé en ce que :
- le groupement éliminable A est constitué par un atome d'halogène de préférence un atome de chlore, de brome ou de fluor ou par un groupement nitro,
- $R'_3$ et $R'_4$ identiques ou différents représentent un atome d'hydrogène, un atome d'halogène, un groupe trifluorométhyl ou nitro,
- $R'_6$ représente un atome d'halogène, un atome d'hydrogène ou un groupement nitro.

3. Procédé de préparation selon la revendication 1 ou 2, caractérisé en ce que :
   - la cyclisation du produit de formule (IV) est avantageuse ment effectuée en présence d'hydrure de lithium au sein d'un solvant organique anhydre.

4. Procédé pour la préparation d'un produit répondant à la formule (I) :

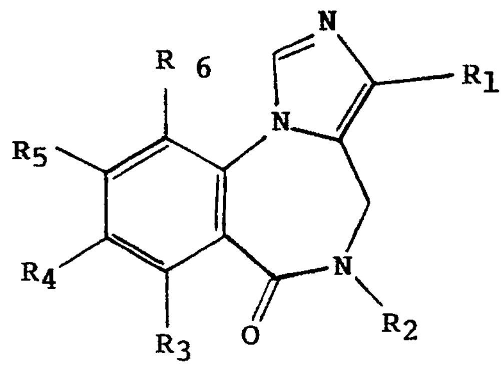

(I)

dans laquelle :

$R_2$ représente un atome d'hydrogène, un radical alkyle renfermant de 1 à 5 atomes de carbone, un radical cycloalkyle renfermant de 3 à 6 atomes de carbone, un groupement phényle, un groupement phényle substitué,

$R_3$, $R_4$, identiques ou différents, représentent un atome d'hydrogène, un atome d'halogène, un groupement $CX_3$ dans lequel X représente un atome d'halogène, un groupement $NO_2$, un groupement CN, un groupement NRR', un groupement CONRR', dans lesquels R représente un atome d'hydrogène ou un radical alkyle renfermant de 1 à 5 atomes de carbone et R' représente un radical alkyle renfermant de 1 à 5 atomes de carbone, ou R et R' ensemble représentent un radical alkylène, renfermant de 2 à 5 atomes de carbone, soit $R_3$ et $R_4$ représentent un radical $OR_7$, $SR_7$, $COOR_7$ ou $COR_7$ dans lequel $R_7$ représente un atome d'hydrogène, un radical alkyle renfermant de 1 à 5 atomes de carbone ou un groupement phényl,

$R_5$ représente un atome d'hydrogène ou de fluor et que :

- soit $R_1$ représente un groupement :

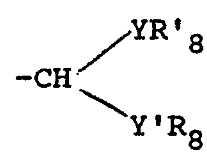

dans lequel $R_8$ et $R'_8$ identiques ou différents représentent un radical alkyle renfermant de 1 à 3 atomes de carbone ou $R_8$ et $R'_8$ ensemble représentent un groupe alkylène contenant de 2 à 5 atomes de carbone et Y et Y' identiques ou différents, représentent un atome d'oxygène ou un atome de soufre, et $R_6$ représente un atome d'hydrogène, un atome d'halogène, un groupement nitro, un groupement amino, un groupement -NRR' dans lequel R et R' ont la signification déjà indiquée ou $R_6$ représente un groupement $SR_7$, un groupement $OR_7$, un groupement $COR_7$, groupements dans lesquels $R_7$ a la signification déjà indiquée,

- soit $R_1$ représente un groupement $COOR_9$ dans lequel $R_9$ représente un radical alcoyle renfermant de 1 à 5 atomes de carbone ou $R_1$ représente un groupement cycloalkyle renfermant de 3 à 6 atomes de carbone, et $R_6$ représente un atome d'hydrogène, un atome d'halogène, un groupement nitro, un groupement NRR', un groupement CONRR' dans lesquels R et R' ont la signification déjà indiquée, un radical $-OR_7$, $SR_7$, $COOR_7$ ou $COR_7$ dans lesquels $R_7$ a la signification déjà indiquée, ou un groupement $-N=CH-OR_9$ dans lequel $R_9$ a la signification déjà indiquée, ou $R_6$ représente un groupement CN, un groupement amino, un groupement $-N=CH-N(CH_3)_2$ ou un groupement thiocyano, isothiocyano ou thioacétyle,

- soit $R_1$ représente un groupement $-COR'_c$ dans lequel $R'_c$ représente cycloalkyle renfermant de 3 à 6 atomes de carbone et $R_6$ représente un atome d'hydrogène, un atome d'halogène, un groupement nitro, un groupement -NRR' ou -CONRR' dans lesquels R et R' ont la signification

déjà indiquée, ou un groupement - $SR_7$, $-OR_7$, $-COOR_7$ ou $-COR_7$ dans lesquels $R_7$ a la signification déjà indiquée, ou un groupement $-N=CH-OR_9$ dans lequel $R_9$ a la signification déjà indiquée, ou un groupement CN, ainsi que leurs sels, procédé caractérisé en ce que l'on fait réagir un produit de formule ($I_B$) :

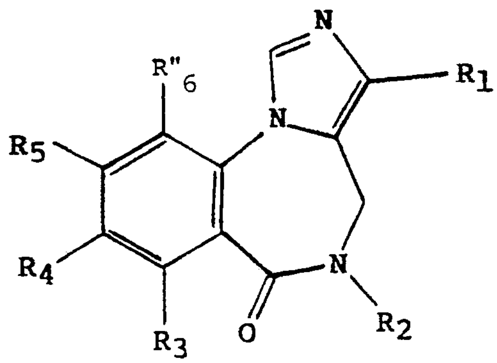

($I_B$)

dans laquelle $R_1$, $R_2$, $R_3$, $R_4$ et $R_5$ ont la signification déjà indiquée et $R''_6$ a l'une des significations indiquées ci-dessus pour $R_6$ à l'exception du groupement $R_6$ désiré, avec un réactif approprié capable de remplacer le groupement $R''_6$ par le groupement $R_6$, et si désiré salifie le produit de formule (I) obtenu.

**5.** Procédé de préparation selon la revendication 4, caractérisé en ce que le produit de formule ($I_B$) est un produit dans lequel $R''_6$ représente un groupe électro-attracteur et qu'il est préparé selon l'une quelconque des revendications 1 à 3.

**6.** Procédé de préparation selon la revendication 4 ou 5, caractérisé en ce que l'on prépare un produit de formule (I) dans laquelle $R_6$ représente un groupe $NH_2$, par réduction d'un produit de formule ($I_B$) dans laquelle $R''_6$ représente un groupe $NO_2$.

**7.** Procédé de préparation selon la revendication 4 ou 5, caractérisé en ce que l'on prépare un produit de formule (I) dans laquelle $R_6$ représente un groupe $-N=CH-O-R_9$ dans lequel $R_9$ est défini comme à la revendication 4, caractérisé en ce que l'on fait réagir un produit de formule ($I_B$) dans lequel $R''_6$ représente un groupement $NH_2$ avec un trialkyl orthoformate au reflux du mélange réactionnel.

**8.** Procédé de préparation selon la revendication 4 ou 5, caractérisé en ce que l'on prépare un produit de formule (I) dans laquelle $R_6$ représente un groupe NRR' dans lequel R et R' sont définis comme à la revendication 4, caractérisé en ce que l'on réduit un produit de formule ($I_B$) dans lequel $R''_6$ représente un groupement $-N=CH-O-R_9$ dans lequel $R_9$ est défini comme à la revendication 4.

**9.** Procédé de préparation selon la revendication 4 ou 5, caractérisé en ce que l'on prépare un produit de formule (I) dans laquelle $R_6$ représente un groupe $-SR_7$ dans lequel $R_7$ est défini comme à la revendication 4, caractérisé en ce que l'on fait réagir un produit de formule ($I_B$) dans lequel $R''_6$ représente un groupement $NH_2$ avec du butyl nitrite puis avec un produit de formule $R_7$-S-Cu.

**10.** Procédé de préparation des produits de formule ($I_C$) :

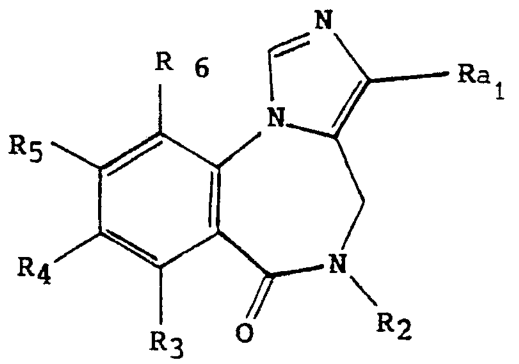

$(I_C)$

dans laquelle $R_2$, $R_3$, $R_4$, $R_5$ et $R_6$ ont la signification déjà indiquée et $Ra_1$ représente un groupe -COR'$_c$ dans lequel R'$_c$ a la signification déjà indiquée à la revendication 4, procédé caractérisé en ce que l'on fait réagir un produit de formule (VIII) :

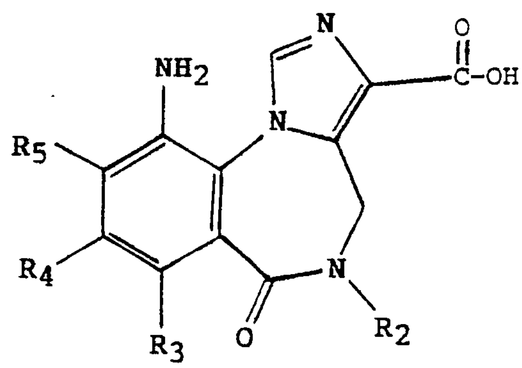

(VIII)

dans laquelle $R_2$, $R_3$, $R_4$ et $R_5$ ont la signification déjà indiquée, avec le 1,1-carbonyldiimidazole pour obtenir un produit de formule (VII) :

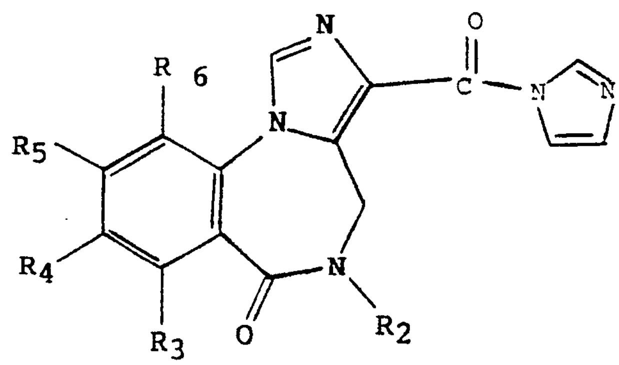

(VII)

dans laquelle $R_2$, $R_3$, $R_4$, $R_5$ et $R_6$ ont la signification déjà indiquée, que l'on fait réagir avec le chlorhydrate de N,O-diméthylhydroxylamine pour obtenir un produit de formule (V) :

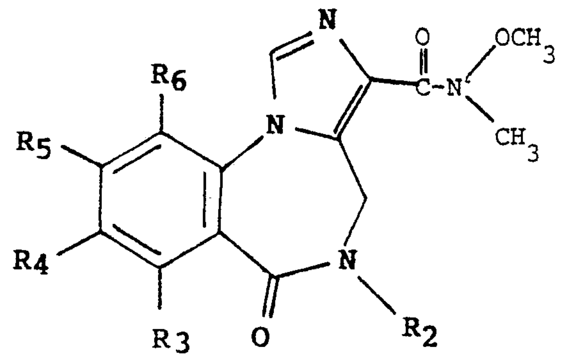

(V)

dans laquelle $R_2$, $R_3$, $R_4$, $R_5$ et $R_6$ ont la signification déjà indiquée, que l'on fait réagir avec un produit de formule (VI) :

$$R'_c\text{-Mg-Hal} \qquad (VI)$$

dans laquelle $R'_c$ a la signification déjà indiquée et Hal représente un atome d'halogène et si désiré, salifie le produit de formule ($I_c$) obtenu.

11. A titre de produits nouveaux, les imidazobenzodiazépines de formule (I), dont les noms suivent :
    - 5,6-dihydro-5-méthyl-10-nitro-6-oxo-4H-imidazo [1,5-a] [1,4] benzodiazépine-3-carboxylate d'éthyle,
    - 5,6-dihydro-10-fluoro-5-méthyl-6-oxo-4H-imidazo [1,5-a] [1,4] benzodiazépine-3-carboxylate d'éthyle,
    - 7,10-difluoro-5,6-dihydro-5-méthyl-6-oxo-4H-imidazo [1,5-a] [1,4] benzodiazépine-3-carboxylate d'éthyle,
    - 9,10-difluoro-5,6-dihydro-5-méthyl-6-oxo-4H-imidazo [1,5-a] [1,4] benzodiazépine-3-carboxylate d'éthyle,
    - 5,6-dihydro-5-méthyl-6-oxo-7,8,9,10-tétrafluoro-4H-imidazo [1,5-a] [1,4] benzodiazépine-3-carboxylate d'éthyle,
    - 5,6-dihydro-5-(4-méthoxyphényl)-6-oxo-4H-imidazo [1,5-a] [1,4] benzodiazépine-3-carboxylate d'éthyle,
    - 5,6-dihydro-5-(4-méthoxyphényl)-6-oxo-4H-imidazo [1,5-a] [1,4] benzodiazépine-3-yl-cyclopropylméthanone,
    - 7-chloro-5,6-dihydro-5-méthyl-6-oxo-4H-imidazo [1,5-a] [1,4] benzodiazépine-3-yl-cyclopropylméthanone,
    - 5,6-dihydro-5-méthyl-6-oxo-7-trifluorométhyl-4H-imidazo [1,5-a] [1,4] benzodiazépine-3-yl-cyclopropylméthanone,
    - 5,6-dihydro-7-fluoro-5-méthyl-6-oxo-4H-imidazo [1,5-a] [1,4] benzodiazépine-3-yl-cyclopropylméthanone,
    - 5,6-dihydro-6-oxo-4H-imidazo [1,5-a] [1,4] benzodiazépine-3-yl-cyclopropylméthanone,
    - 7-chloro-3-diéthyloxyméthyl-5,6-dihydro-5-méthyl-4H-imidazo [1,5-a] [1,4] benzodiazépine-6-oxo (4H)-one,
    - 5,6-dihydro-10-amino-5-méthyl-6-oxo-4H-imidazo [1,5-a] [1,4] benzodiazépine-3-carboxylate d'éthyle,
    - 5,6-dihydro-10-éthoxyméthylèneamino-5-méthyl-6-oxo-4H-imidazo [1,5-a] [1,4] benzodiazépine-3-carboxylate d'éthyle,
    - 5,6-dihydro-10-(diméthylamino-méthylèneamino)-5-méthyl-6-oxo-4H-imidazo [1,5-a] [1,4] benzodiazépine-3-carboxylate d'éthyle,
    - 5,6-dihydro-5-méthyl-10-méthylamino-6-oxo-4H-imidazo [1,5-a] [1,4] benzodiazépine-3-carboxylate d'éthyle,
    - 5,6-dihydro-5-méthyl-10-méthylthio-6-oxo-4H-imidazo [1,5-a] [1,4] benzodiazépine-3-carboxylate d'éthyle,

- 5,6-dihydro-5-méthyl-10-thiocyano-6-oxo-4H-imidazo [1,5-a] [1,4] benzodiazépine-3-carboxylate d'éthyle,
- 5,6-dihydro-10-isothiocyano-5-méthyl-6-oxo-4H-imidazo [1,5-a] [1,4] benzodiazépine-3-carboxylate d'éthyle,
- 5,6-dihydro-10-iodo-5-méthyl-6-oxo-4H-imidazo [1,5-a] [1,4] benzodiazépine-3-carboxylate d'éthyle,
- 5,6-dihydro-5-méthyl-10-thioacétyl-6-oxo-4H-imidazo [1,5-a] [1,4] benzodiazépine-3-carboxylate d'éthyle, ainsi que leurs sels d'addition avec les acides.

**12.** Procédé de préparation selon l'une quelconque des revendications 1 à 10, pour la préparation des produits, dont les noms suivent :
- 5,6-dihydro-5-méthyl-7-nitro-6-oxo-4H-imidazo [1,5-a] [1,4] benzodiazépine-3-carboxylate d'éthyle,
- 5,6-dihydro-5-méthyl-8-nitro-6-oxo-4H-imidazo [1,5-a] [1,4] benzodiazépine-3-carboxylate d'éthyle,
- 7-chloro-5,6-dihydro-5-méthyl-6-oxo-4H-imidazo [1,5-a] [1,4] benzodiazépine-3-carboxylate d'éthyle,
- 5,6-dihydro-5-méthyl-6-oxo-7-trifluorométhyl-4H-imidazo [1,5-a] [1,4] benzodiazépine-3-carboxylate d'éthyle,
- 5,6-dihydro-7-iodo-5-méthyl-6-oxo-4H-imidazo [1,5-a] [1,4] benzodiazépine-3-carboxylate d'éthyle,
- 5,6-dihydro 7-fluoro 5-méthyl 6-oxo 4H-imidazo [1,5-a] [1,4] benzodiazépine-3-carboxylate d'éthyle, ainsi que leurs sels d'addition avec les acides minéraux ou organiques.

**13.** Médicaments, caractérisés en ce qu'ils sont constitués par les nouvelles imidazobenzodiazépines de formule (I) telle que définies à la revendication 11.

**14.** Médicaments, caractérisés en ce qu'ils sont constitués par les imidazobenzodiazépines dont les noms suivent :
- 7-chloro-5,6-dihydro-5-méthyl-6-oxo-4H-imidazo [1,5-a] [1,4] benzodiazépine-3-yl-cyclopropylméthanone,
- 5,6-dihydro-5-méthyl-6-oxo-7-trifluorométhyl-4H-imidazo [1,5-a] [1,4] benzodiazépine-3-yl-cyclopropylméthanone,
- 5,6-dihydro-7-fluoro-5-méthyl-6-oxo-4H-imidazo [1,5-a] [1,4] benzodiazépine-3-yl-cyclopropylméthanone.

**15.** Compositions pharmaceutiques, caractérisées en ce qu'elles renferment à titre de principe actif, l'un au moins des médicaments tels que définis à la revendication 13 ou 14.

**16.** A titre de produits industriels nouveaux :
- les produits de formule (IV) :

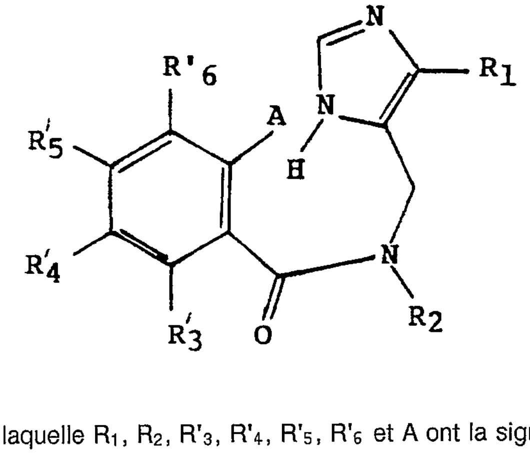

(IV)

dans laquelle $R_1$, $R_2$, $R'_3$, $R'_4$, $R'_5$, $R'_6$ et A ont la signification indiquée à la revendication 1.
- les produits de formule (V) :

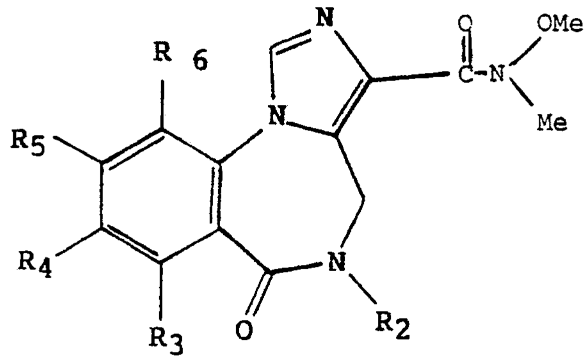

(V)

dans laquelle $R_2$, $R_3$, $R_4$, $R_5$ et $R_6$ ont la signification indiquée à la revendication 10 à l'exception des produits de formule (V') :

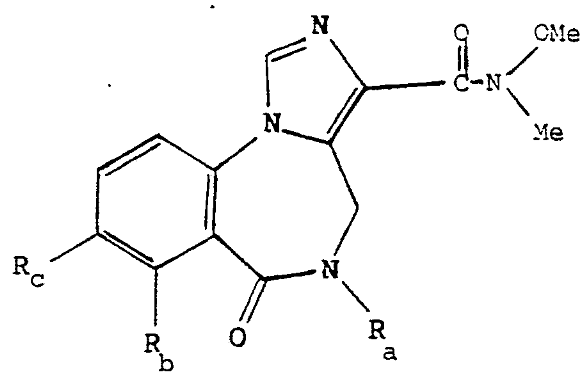

(V')

dans laquelle $R_b$ et $R_c$, identiques ou différents, représentent un atome d'hydrogène, un atome d'halogène, un groupe nitro, nitrile ou trifluorométhyle, $R_a$ représente un atome d'hydrogène, un radical alkyle renfermant de 1 à 5 atomes de carbone ou un groupe cycloalkyle renfermant de 3 à 5 atomes de carbone.

- Les produits de formule (VII) :

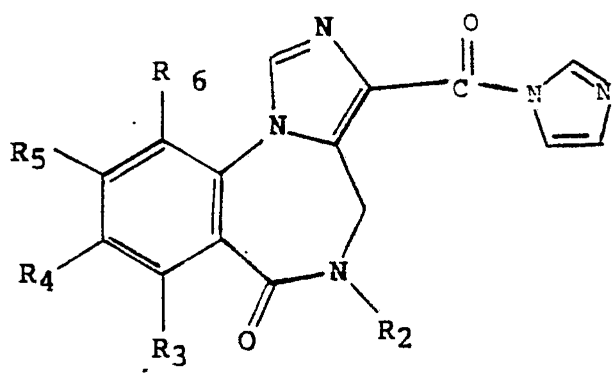

(VII)

dans laquelle $R_2$, $R_3$, $R_4$, $R_5$ et $R_6$ ont la signification indi quée à la revendication 10, à l'exception des produits de formule (VII') :

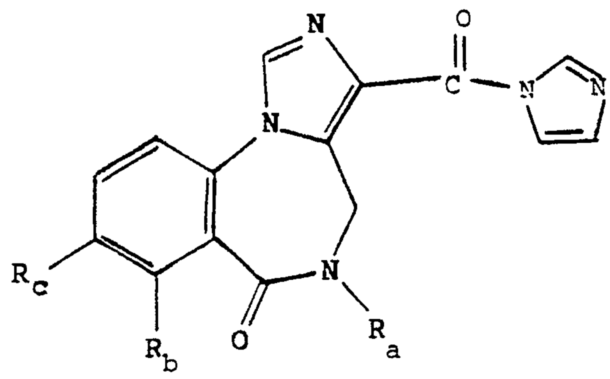

(VII')

dans laquelle $R_a$, $R_b$ et $R_c$ ont la signification indiquée ci-dessus.